# EUROPEAN PATENT APPLICATION

(11) **EP 4 190 319 A1**
(43) Date of publication of application: **07.06.2023**
(21) Application number: 20947806.4
(22) Date of filing: 29.07.2020
(51) Int. Cl.: A61K 31/13, A61K 31/495, A61K 31/685, A61K 47/38, A61K 47/04, A61K 9/28, A61P 25/00, A61P 25/16

(54) **PHARMACEUTICAL COMPOSITION COMPRISING MEMANTINE AND CITICOLINE**

(71) Applicant: Novamedica Limited Liability Company, Moscow 125196 (RU)
(72) Inventor: IXANOV, Rustam Munirovich, Moscow, 117393 (RU); LITVINOVA, Elena Yurievna, Moscow, 125080 (RU); LEYKIN, Zakhar Naumovich, Moscow, 117574 (RU); KUZNETSOVA, Irina Gennadievna, Moscow, 109428 (RU); SALAKHETDINOV, Damir Khizbullaevich, Moscow, 127055 (RU); ROGOZHKINA, Elena Alekseevna, g. Lyubertsy, 140002 (RU)
(74) Representative: Michalski Hüttermann & Partner Patentanwälte mbB
(86) International application number: PCT/RU2020/000389
(87) International publication number: WO 2022/025785

(57) **Abstract**

The invention relates to a pharmaceutical composition having a neuromodulatory, antiparkinsonism, neuroprotective, and antispastic effect and intended for the treatment of cognitive disorders of vascular origin, and comprising memantine or its pharmaceutically acceptable salt and citicoline or its pharmaceutically acceptable salt, as well as a method of producing a dosage form based on the pharmaceutical composition. The therapeutic activity of citicoline or its pharmaceutically acceptable salt is increased because of a synergistic effect when citicoline or its pharmaceutically acceptable salt is used concomitantly with memantine or its pharmaceutically acceptable salt.

## Description

### FIELD OF APPLICATION

The proposed inventions relate to the field of pharmaceutics and, in particular, to pharmaceutical compositions and solid oral dosage forms having neuromodulatory, antiparkinsonism, neuroprotective and antispastic effects and slowing down and inhibiting glutamargic neurotransmission and progress of neurodegenerative processes, methods for producing such pharmaceutical compositions and solid oral dosage forms, and use of said pharmaceutical compositions and solid oral dosage forms for therapy of cognitive disorders of vascular origin.

### PRIOR ART

In the modem market of pharmaceutical preparations, the sector representing the development of various dosage forms used, primarily, for therapy of cognitive disorders of vascular origin is topical and increasingly demanded; said therapy is directed to improvement of memory, prevention and treatment of cerebral ischemia, treatment of disorders of the nervous system, consequences of a traumatic brain injury, ischemic stroke and hemorrhagic stroke, prevention and treatment of diabetic neuropathy as well as to treatment of all kinds of dementia, including the Alzheimer's disease, from a mild to moderate severity level; and the development of drug products having neuromodulatory, antiparkinsonism, neuroprotective and antispastic effects and slowing down or inhibiting glutamargic neurotransmission and progress of neurodegenerative processes.

It is well known that such substance as citicoline (cytidine diphosphate-choline, cytidine 5'-diphosphocholine, CDP-choline, Neurocoline), or 5'-O-[hydroxy({hydroxy[2-(trimethylammonio)ethoxy]phosphoryl}oxy)phosphoryl]cytidine - a substance of formula (I) with nootropic action - is rather actively used for developing modern drug products intended for prevention and treatment of the above therapeutic disorders; this substance is a natural endogenous compound that is an intermediate metabolite in the synthesis of phosphatidylcholine - one of the main structural components of the cell membrane.

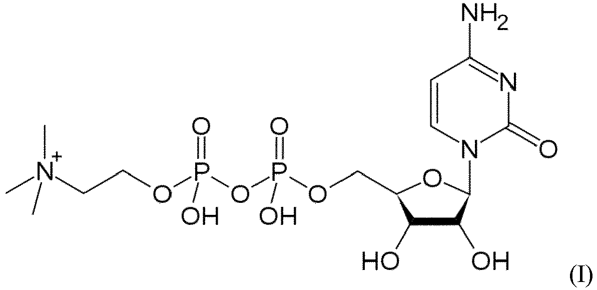

The invention [1] disclosed in Patent RU2429851, publ. 27.09.2011, is known in the art, wherein a method for increasing intelligence of a subject is described, which consists in administering a pharmaceutical composition comprising (a) a polyunsaturated fatty acid selected from an omega-3 fatty acid or omega-6 fatty acid, (b) uridine, and (c) a choline salt. In particular embodiments of the invention [1], uridine is an acyl derivative of uridine, uridine phosphate or cytidine 5'-diphosphocholine (CDP-choline). The patent [1] further discloses a pharmaceutical composition for increasing intelligence of a subject, which comprises: (a) a polyunsaturated fatty acid selected from an omega-3 fatty acid or omega-6 fatty acid, (b) uridine, and (c) a choline salt; and in particular embodiments of the invention [1], uridine is an acyl derivative of uridine, uridine phosphate or cytidine 5'-diphosphocholine (CDP-choline).

The invention, disclosed in patent RU 2603470, publ. 27.11.2016 [2], is also known in the art, which relates to a method for improving learning ability or memory of a subject, comprising administering a pharmaceutical composition comprising (a) an omega-3 fatty acid or omega-6 fatty acid or a combination thereof, (b) uridine, its acyl derivative, uridine phosphate or cytidine 5'-diphosphocholine (CDP-choline), and (c) choline, its metabolic precursor or a choline salt to the subject. Claim 6 clarifies that uridine is an acyl derivative of uridine, uridine phosphate or cytidine 5'-diphosphocholine (CDP-choline).

Further, the patent [2] discloses a pharmaceutical composition for improving learning ability or memory of a subject, comprising (a) an omega-3 fatty acid or omega-6 fatty acid or a combination thereof, (b) uridine, and (c) a choline salt. In particular embodiments of the invention [2], uridine is an acyl derivative of uridine, uridine phosphate or cytidine 5'-diphosphocholine (CDP-choline).

The analogs [1] and [2] disclose pharmaceutical compositions that provide intelligence improvement in a subject due to the synergistic action of the components of the composition comprising CDP-choline (citicoline) in relation to an increase in a concentration of the brain phospholipids, but they do not act on the glutamatergic system. On the contrary, the composition proposed in the present invention, unlike the technical solutions [1] and [2], provides a therapeutic effect not only by increasing a concentration of the brain phospholipids, but also by providing a modulating action to the glutamatergic system.

Furthermore, the invention of patent RU2605339, publ. 20.12.2016 [3] is known in the art, which discloses a therapeutic agent based on amantadine sulphate for reduction of glutamate-induced apoptosis and inhibition of NMDA-receptors for treating disorders of the nervous system, consequences of a traumatic brain injury and ischemic stroke and hemorrhagic stroke, and is characterized in that it comprises, as the active substance, a therapeutically effective quantity of amantadine sulphate and cytidine 5'-diphosphocholine, as well as at least one adjuvant selected from the group comprising stabilizers, prolongators, buffering additives, solvents, excipients, and preservatives, the component ratio being as follows, wt%:
amantadine sulphate - 0.001-0.25
cytidine 5'-diphosphocholine - 5.0-25.0
adjuvant - up to 100.

This patent also discloses the use of that therapeutic agent for reduction of glutamate-induced apoptosis and inhibition of NMDA-receptors for treating disorders of the nervous system, consequences of a traumatic brain injury and ischemic stroke and hemorrhagic stroke.

The analog [3] discloses a therapeutic agent for reduction of glutamate-induced apoptosis and inhibition of NMDA-receptors. The therapeutic agent according to the analog [3] does not have an effect on a concentration of phospholipids in the brain and the spinal cord. On the contrary, the solution claimed in the present invention, unlike the therapeutic agent according to the analog [3], provides an additional therapeutic effect due to increasing a concentration of phospholipids in the brain and the spinal cord.

As the second active substance of the present invention, memantine or 3,5-dimethyladamantan-1-amine is selected that is a noncompetitive antagonist to glutamate NMDA-receptors of the formula (II), exhibits neuromodulatory, antiparkinsonism, neuroprotective and antispastic effects, and inhibits glutamatergic neurotransmission and progress of neurodegenerative processes.

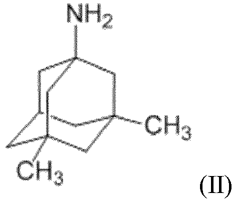

Memantine (II) may be also used in the form of its pharmaceutically acceptable salt, e.g. in the form of memantine hydrochloride (hydrochloride salt).Thus, patent RU2371173, publ. 27.10.2009 [4], teaches a method for treating the Alzheimer's disease of various severity, from mild to moderate, which consists in administering an effective quantity of memantine or its pharmaceutically acceptable salt, such as its hydrochloride salt, to a subject in need thereof, memantine or its pharmaceutically acceptable salt, according to [4], being administered in the initial dose of 5 mg/day which is increased by 5 mg/day every week, until the final dose of 20 mg/day is reached. Also, patent [4] teaches a method for treating the Alzheimer's disease of mild to moderate severity in subjects who are treated with acetylcholinesterase inhibitor (AChEI), and the method consists in administering an effective quantity of memantine or its pharmaceutically acceptable salt to a subject in need thereof. Patent [4] discloses a method for treating one form of demetia (Alzheimer's disease) only by using memantine or its pharmaceutically acceptable salt, but does not imply the use of memantine or its pharmaceutically acceptable salt for treating other forms of dementia (in particular, vascular dementia, as in the claimed solution).

The invention disclosed in Patent RU2326660, publ. 20.06.2008 [5], is known in the art, which discloses an oral drug product in the solid dosage form comprising memantine as the active ingredient, and which is characterized in that the dosage form is made as capsules, and memantine is included in a therapeutically effective quantity into the composition of the mass contained in the capsules, which is a mixture of powders.

Also, the patent [5] discloses:
- an oral drug product made as a solid dosage form comprising memantine as the active ingredient, which is characterized in that the dosage form is made as capsules, and memantine is included in a therapeutically effective quantity into the composition of the mass contained in the capsules, which is a granulated material;
- a method for producing the oral drug product in a solid dosage form comprising memantine as the active ingredient, the method consisting in that powders of memantine and excipients - a desintegrant, an antifriction agent and a vehicle - are sieved, weighed and mixed in the following component ratios, in wt%:

| | |
|---|---|
| memantine | 0.5-10.0 |
| desintegrant | 0.2-40.0 |
| antifriction agent | 0.2-10.0 |
| vehicle | the rest, |

then, capsules are filled with the produced mass in the form of a mixture of powders;
- a method for producing the oral drug product in a solid dosage form comprising memantine as the active ingredient, which consists in that an vehicle and, when necessary, a desintegrant and an antifriction agent are used as excipients, and powders of memantine and the vehicle or, when necessary, powders of the desintegrant and the antifriction agent are also sieved, weighed and mixed in the following component ratios, in wt%:

| | |
|---|---|
| memantine | 0.5-10.0 |
| vehicle | the rest, |

or

| | |
|---|---|
| memantine | 0.5-10.0 |
| desintegrant | 0.2-40.0 |
| antifriction agent | 0.2-10.0 |
| vehicle | the rest, |

then the produced mixture of powders is granulated by a dry or wet granulation method, and capsules are filled with the granulated material.

The invention disclosed in Patent RU 2404750 [6], publ. 27.11.2010, is also known in the art, which relates to a pharmaceutical composition comprising memantine or its pharmaceutically acceptable salt and intended for use in treatment of dementias, wherein said memantine or its pharmaceutically acceptable salt are present in a prolonged-release dosage form; the composition has to be administered without using regimen with dose increase; to a pharmaceutical composition comprising memantine or its pharmaceutically acceptable salt and a prolonged-release component for the use in treatment of dementias, where said treatment results in a therapeutically effective, permanent concentration of memantine in plasma within 20 days from the time of administering the composition to a patient; to a pharmaceutical composition in the form of a capsule comprising memantine or its pharmaceutically acceptable salt, where said memantine or its pharmaceutically acceptable salt having a Cₘₐₓ/Cₘₑₐₙ ratio from app. 2.5 to app. 1 for at least 6 hours after administration of the composition to a patient; to a pharmaceutical composition comprising a prolonged-release oral preparation that comprises:
a) memantine and a polymer selected from hydroxypropyl cellulose, polyvinylpyrrolidon, hydroxypropyl methylcellulose and polyethylene glycol; and
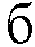 ) a coating comprising an insoluble matrix polymer and a water-soluble substance.

The invention disclosed in Patent RU2483715 [7], publ. 10.06.2013, is also known in the art, which relates to an agent having the antiparkinsonism effect and to a solid, quickly disintegrating dosage form, wherein said dosage form comprises memantine and/or memantine hydrochloride and target additives and is characterized in that said dosage form comprises cellulose II as the target additive, the component ratio being as follows, wt%:

| | |
|---|---|
| memantine and/or memantine hydrochloride | 5-10 |
| cellulose II | 90-95. |

The analogs [5], [6], and [7] describe mono-drug products of memantine or its pharmaceutically acceptable salt - memantine hydrochloride. At the same time, in a number of cases the use of memantine or its pharmaceutically acceptable salt as a single therapeutic agent may be insufficient in order to achieve an effect in a plurality of cognitive disorders caused by other forms of dementia, apart from vascular dementias.

The invention disclosed in Patent RU2390354 [8], publ. 27.05.2010, is also known in the art, which relates to a matrix-type sustained-release composition, comprising:
(1) donepezil hydrochloride and/or memantine hydrochloride;
(2) an enteric polymer which is at least one selected from the group comprising a methacrylic acid-ethyl acrilate copolymer, a metacrylic acid-methyl metacrilate, hydroxypropyl methylcellulose phthalate, and hydroxypropyl methylcellulose acetate succinate; and
(3) a water-insoluble polymer which is at least one selected from the group comprising ethyl cellulose, a RS aminoalkyl metacrilate copolymer, and a ethylacrylate-methylmetacrilate copolymer.

The patent [8] also discloses a method for producing a matrix-type composition with sustained release, comprising the following steps:
mixing: (1) donepezil hydrochloride and/or memantine hydrochloride; (2) an enteric polymer which is at least one selected from the group comprising a methacrylic acid-ethyl acrilate copolymer, a metacrylic acid-methyl metacrilate, hydroxypropyl methylcellulose phthalate, and hydroxypropyl methylcellulose acetate succinate; and (3) a water-insoluble polymer which is at least one selected from the group comprising ethyl cellulose, an RS aminoalkyl metacrilate copolymer, and a ethylacrylate-methylmetacrilate copolymer; and
compression forming of a mixture produced in the step of mixing.

According to Patent [8], memantine hydrochloride is mentioned as an agent used in treatment of a dementia caused by the Alzheimer's disease (see Patent [8], p. 15 of the specification, Section "Best Mode for Carrying Out the Invention"). At the same time, other application fields of memantine and its pharmaceutically acceptable salts are also known, in particular, vascular dementia (Cerebrovascular disease, cognitive impairment and dementia. - in: Cerebrovascular disease and dementia (Second edition) / Edited by J. O'Brien, D. Ames, L. Gustafson et al. London: Martin Dunitz, 2004).

After analyzing the market of drug products intended for therapy of cognitive disorders of vascular origin, which have neuromodulatory, antiparkinsonism, neuroprotective, and antispastic effects, slow down and inhibit glutamatergic neurotransmission and progress of neurodegenerative processes, it becomes evident that it is necessary to develop a pharmaceutical composition and a dosage form based thereon, which would provide an increase in the therapeutic activity of the claimed quantitative and qualitative formulation of the ingredients (a pharmaceutical composition), would be effective, when administered to a patient in need thereof, for therapy of cognitive disorders of vascular origin as well as would have neuromodulatory, antiparkinsonism, neuroprotective, and antispastic effects, slow down and inhibit glutamatergic neurotransmission and progress of neurodegenerative processes.

The technical effects, achieved by the claimed group of the inventions, include:
- an increase in the therapeutic activity of citicoline or its pharmaceutically acceptable salt due to an over-cumulative (synergetic) effect when citicoline or its pharmaceutically acceptable salt is used in combination with memantine or its pharmaceutically acceptable salt as well as due to the instantaneous beginning of action and subsequent effective and sustained action of the proposed pharmaceutical composition and dosage form;
- simplification of therapy while treating cognitive disorders of vascular origin owing to the use of the pharmaceutical composition and a dosage form comprising this composition proposed in the present invention;
- provision of the pharmaceutical composition and dosage form of memantine or its pharmaceutically acceptable salt and citicoline or its pharmaceutically acceptable salt, which have no side effects and are stable during manufacture and storage.

Furthermore, the technical effects, achieved by the claimed group of inventions, include:
- an increase in the therapeutic activity of the pharmaceutical composition and a drug product intended for therapy of cognitive disorders of vascular origin, which have neuromodulatory, antiparkinsonism, neuroprotective, and antispastic effects, slow down and inhibit glutamatergic neurotransmission and progress of neurodegenerative processes due to the synergetic effect achieved owing to the joint use of memantine or its pharmaceutically acceptable salt and citicoline or its pharmaceutically acceptable salt as well as owing to the instantaneous beginning of action of this drug product and its subsequent effective and sustained action;
- simplification of a regimen of treatment (correction, alleviation or deceleraion of development) of cognitive disorders of vascular origin,
- and simplification and a cost reduction of production of the dosage form and the pharmaceutical composition, which are intended for therapy of cognitive disorders of vascular origin, have neuromodulatory, antiparkinsonism, neuroprotective, and antispastic effects, slow down and inhibit glutamatergic neurotransmission and progress of neurodegenerative processes.

Furthermore, the technical effect also consists in expanding the range of pharmaceutical compositions and dosage forms intended for therapy of cognitive disorders of vascular origin, which have neuromodulatory, antiparkinsonism, neuroprotective, and antispastic effects, and slow down and inhibit glutamatergic neurotransmission and progress of neurodegenerative processes.

### SUMMARY OF THE INVENTION

The above technical effects are achieved by the **pharmaceutical composition** that is proposed in this invention and intended for therapy of cognitive disorders of vascular origin, has neuromodulatory, antiparkinsonism, neuroprotective, and antispastic effects, slows down and inhibits glutamatergic neurotransmission and progress of neurodegenerative processes, comprises from 5.00 to 20.00 mg of memantine or its pharmaceutically acceptable salt and from 450 to 550 mg of citicoline or its pharmaceutically acceptable salt; and, further, **the solid oral dosage form,** as proposed in this invention, which **is produced on the basis of the above pharmaceutical composition** and comprises, as the active substances, memantine or its pharmaceutically acceptable salt in the rnage from 5.00 to 20.00 mg and citicoline or its pharmaceutically acceptable salt in a the range from 450 to 550 mg, and is produced in the form of a modified-release tablet coated with a film coating. **The technical effect, which relates to simplification and cost reduction of the dosage form, is achieved by a method, as proposed in this invention, for producing of the solid oral dosage form based on the above pharmaceutical composition.**

The pharmaceutical composition and the dosage form propoed in this invention also comprise, apart from memantine or its pharmaceutically acceptable salt and citicoline or its pharmaceutically acceptable salt, auxiliary components including a pharmaceutically acceptable excipient, hydroxypropyl methylcellulose, colloidal silica and a pharmaceutically acceptable lubricant.

In preferable (not limiting) embodiments of the invention, the pharmaceutically acceptable excipient is selected from microcrystalline cellulose, starch, glucose, sucrose, lactose, basic magnesium carbonate, gelatin, methylcellulose, sodium-carboxymethylcellulose, calcium carbonate, glycine, dextrin, sorbite, and mannitol. In the most preferable embodiment of the invention, the pharmaceutically acceptable excipient is microcrystalline cellulose.

Further, in preferable (not limiting) embodiments of the invention, the pharmaceutically acceptable lubricant is a pharmaceutically acceptable salt of stearic acid, for example (but without limitation), magnesium stearate or calcium stearate. A person skilled in the art will appreciate that another pharmaceutically acceptable lubricant may be used, including another pharmaceutically acceptable salt of stearic acid.

In accordance with this invention, memantine may be used in the form of the base or in the form of its pharmaceutically acceptable salt. In particular (not limiting) embodiments of this invention, memantine may be also used in the form of a pharmaceutically acceptable salt of an inorganic acid, for example in the form of memantine hydrochloride, memantine hydrobromide, memantine sulfate, memantine hydrogen sulfate and memantine dihydrogen sulfate, etc. In other (not limiting) embodiments of this invention, memantine may be used in the form of a pharmaceutically acceptable salt of an organic acid, for example in the form of memantine oxalate, memantine citrate, memantine succinate, memantine maleate, memantine fumarate, memantine malate, memantine tartrate etc., or in the form of sulfonate, for example (but without limitation), in the form of memantine methanesulfonate (mesylate), memantine benzenesulfonate (besilate) or memantine toluenesulfonate (tosylate). In other particular (not limiting) embodiments of this invention, memantine may be also used in the form of memantine hydroiodide, memantine perchlorate, memantine acetate, memantine propionate, memantine glycolate, memantine lactate, memantine pyruvate, memantine malonate, memantine benzoate, memantine carbonate, memantine cinnamate, memantine mandelate, memantine ethane sulfonate, memantine hydroxyethane sulfonate, memantine p-toluene sulfonate, memantine cyclohexane sulfamate, memantine salicylate, memantine p-aminosalicylate, memantine 2-phenoxybenzoate or memantine 2-acetoxybenzoate.

Most preferably (but without limitation), in accordance with this invention, memantine is used in the form of memantine hydrochloride.

In accordance with this invention, citicoline *per se,* may be used in the form of the base or in the form of its pharmaceutically acceptable salt. In particular (not limiting) embodiments of this invention, citicoline may be also used in the form of a pharmaceutically acceptable salt of an inorganic acid, such as hydrochloric acid or phosphoric acid, or in the form of a pharmaceutically acceptable salt of an organic acid, such as acetic acid, oxalic acid, tartaric acid, mandelic acid, etc. as well as in the form of a pharmaceutically acceptable salt formed by free carboxy groups with inorganic ions, including ions of sodium, potassium, ammonium, calcium, iron hydroxides, as well as with organic bases, such as isopropyl amine, trimethyl amine, 2-ethyl aminoethanol, histidine, procaine, etc.

Most preferably (but without limitation), in accordance with this invention, citicoline is used in the form of the citicoline monosodium salt (also referred to herein as citicoline monosodium). According to the method proposed in this invention, a lubricant, citicoline or its pharmaceutically acceptable salt, memantine or its pharmaceutically acceptable salt, a pharmaceutically acceptable excipient, hydroxypropyl methylcellulose, colloidal silica are all sifted through a sieve with a mesh diameter not more than 0.5 mm; then, the citicoline monosodium salt, microcrystalline cellulose and hydroxypropyl methylcellulose are loaded into a mixer-granulator or a similar mixing device and are mixed to a homogenous state at a mixing rate from 160 to 240 rpm and for a period from 2 to 10 minutes; further, the obtained mixture is moistened with purified water at constant stirring, the stirring rate being from 150 to 400 rpm, and a period of stirring should be not more than 5 minutes; then, the obtained granulated material is dried in the fluidized-bed conditions, while maintaining the following parameters: an input air temperature - 45-65°C, residual moisture of the granulated material - from 2.5 to 4.5%; the obtained granulated material is sifted through a sieve with a mesh diameter of not more than 1.0 mm; then, the obtained mixture is dusted with colloidal silica for 3-5 minutes; then, a portion of hydroxypropyl methylcellulose is added to the mixture; it is mixed once for another 8-10 minutes; then, magnesium stearate is added, and the mixture is mixed for another 2-3 minutes. The obtained tableting mixture is pressed in a rotary tableting press with the use of a tablet tooling having a diameter from 10 to 15 mm; the following parameters of the tableting process being monitored: hardness - 150-290 H, grateness - not more than 1.0%, height - not more than 7.0 mm; after that, a film coating I is applied to the obtained tablet cores, the coating being a mixture of hydroxypropyl methylcellulose, titanium dioxide and macrogol; in the process of dissolving the film coating I, a pharmaceutical substance of memantine hydrochloride is added in portions and is mixed until formation of a homogenous suspension, after that, the obtained coating is applied with the tablet cores temperature of 35-40°C and with intermittent mixing, including that in the drum of a coater; then, a solution of a film coating II is prepared from a mixture of polyvinyl alcohol, talc, macrogol, iron oxides and titanium dioxide upon preparation of a film-forming solution, thus the film coating II is produced that is applied to the tablets over the film coating I in the same conditions as used for the film coating I.

In particular preferable (not limiting) embodiments of the invention, the film coating I and/or the film coating II may also comprise the active substances selected from memantine or its pharmaceutically acceptable salt and citicoline or its pharmaceutically acceptable salt. In the most preferable embodiment of the invention, the film coating I comprises a memantine pharmaceutically acceptable salt, e.g. memantine hydrochloride, and the film coating II comprises a citicoline pharmaceutically acceptable salt, e.g., the citicoline monosodium salt. In another preferable embodiment of the invention, the film coating I, on the contrary, comprises a citicoline pharmaceutically acceptable salt, e.g., the citicoline monosodium salt, and the film coating II comprises a memantine pharmaceutically acceptable salt, e.g., memantine hydrochloride.

In a preferable (not limiting) embodiment of the invention, microcrystalline cellulose is a commercially available microcrystalline cellulose of 101 grade. Further, in a preferable (not limiting) embodiment of the invention, hydroxypropyl methylcellulose is hydroxypropyl methylcellulose with the molecular weight of 1,000,000 Da, e.g. (but without limitation), hypromellose of 2208 type (K100M) or similar. Finally, in a preferable (not limiting) embodiment of the invention, colloidal silica is colloidal silica of A 200 grade.

The technical effects are achieved by this invention owing to that a **pharmaceutical composition** is proposed that provides an increase in therapeutic activity of a quantitative and qualitative formulation of ingredients, is effective in therapy of cognitive disorders of vascular origin, has neuromodulatory, antiparkinsonism, neuroprotective, and antispastic effects, slows down and inhibit glutamatergic neurotransmission and progress of neurodegenerative processes, comprises from 5 to 20 mg of memantine or its pharmaceutically acceptable salt and from 450 to 550 mg of citicoline or its pharmaceutically acceptable salt. An illustrative quantitative and qualitative example of the pharmaceutical composition formulation is presented in Table 1. Other illustrative examples of the quantitative and qualitative formulation of the ingredients of the pharmaceutical composition are presented in Table 2. A person skilled in the art will appreciate that these examples are illustrative (not limiting) only, and are provided for the purposes of illustrating this invention, rather than for limiting the scope of claims.

**Table 1. Quantitative and qualitative formulation of the pharmaceutical composition**

| Components | Content, % | Content, mg |
|---|---|---|
| Citicoline monosodium salt | 65.95-69.95 | 450-550 |
| Memantine hydrochloride | 0.67-2.50 | 5-20 |
| Microcrystalline cellulose | 0.63-5.13 | 5-35 |
| Hydroxypropyl methylcellulose | 17.23-17.80 | 120.5-140.6 |
| Colloidal silica | 0.91-1.0 | 6.8-7.2 |
| Magnesium stearate | 0.91-1.0 | 6.8-7.2 |
| Film coating I | 5.18-5.33 | 36.4-41.5 |
| Film coating II | 2.85-3.2 | 21.8-24.5 |

In a preferable (not limiting) embodiment of the invention, microcrystalline cellulose is microcrystalline cellulose of 101 grade. A person skilled in the art will appreciate that other microcrystalline cellulose variations having similar characteristics may be also used, e.g., microcrystalline cellulose of 105, 102, 103, 302, 200, 50 M, 90 M grades or similar.

Preferably (but without limitation), hydroxypropyl methylcellulose is hydroxypropyl methylcellulose with the molecular weight of 1,000,000. Most preferably (but also without limitation), hydroxypropyl methylcellulose is hypromellose of 2208 type (K100M). A person skilled in the art will appreciate that other hydroxypropyl methylcellulose variations having similar characteristics may be also used, e.g., hypromellose of 2208 K100M DC, K200M types, or similar.

Further, in a preferable (not limiting) embodiment of the invention, colloidal silica is colloidal silica of A 200 grade. A person skilled in the art will appreciate that other colloidal silica variations having similar characteristics may be also used, e.g., colloidal silica of A 300, A 380, R 972 grades, or similar.

Further, in a preferable (not limiting) embodiment of the invention, the film coating I is produced from a commercially available mixture of hydroxypropyl methylcellulose, titanium dioxide and macrogol. In the most preferable (not limiting) embodiment of the invention, the film coating I is produced from the commercially available Opadry 03F180011 white mixture. A person skilled in the art will appreciate that other commercially available mixtures intended for producing film coatings, which have similar characteristics, may be also used, e.g., Opadry based on hydroxypropyl methylcellulose, which include other or similar auxiliary components, as well as Opadry II based on hydroxypropyl methylcellulose, which include other or similar auxiliary components.

Further, in a preferable (not limiting) embodiment of the invention, the film coating II is produced from a commercially available mixture of polyvinyl alcohol, talc, macrogol, iron oxides, and titanium dioxide.

In the most preferable (not limiting) embodiment of the invention, the film coating II is produced from the commercially available Opadry II 85F 200062 purple mixture. A person skilled in the art will appreciate that other commercially available mixtures intended for producing film coatings, which have similar characteristics, may be also used, e.g., Opadry based on polyvinyl alcohol, which includes other auxiliary or similar components, as well as Opadry II based on hydroxypropyl methylcellulose, which includes other auxiliary components, or similar.

**The above technical effects are also achieved by this invention owing to that the pharmaceutical composition is proposed,** which provides an increase in therapeutic activity of the quantitative and qualitative formulation of the ingredients, is effective in therapy of cognitive disorders of vascular origin, has neuromodulatory, antiparkinsonism, neuroprotective, and antispastic effects, slows down and inhibits glutamatergic neurotransmission and progress of neurodegenerative processes, and is produced in the form of a tablet coated with a film coating and comprising from 5 to 20 mg of memantine hydrochloride and from 450 to 550 mg of the citicoline monosodium salt, as well as adjuvants including microcrystalline cellulose, hydroxypropyl methylcellulose with the molecular weight of 1,000,000, colloidal silica, and magnesium stearate, the ratio of the quantitative and qualitative formulation of the ingredients of the pharmaceutical composition being as follows, wt%:

| | |
|---|---|
| Citicoline monosodium salt | 65.95-69.95 |
| Memantine hydrochloride | 0.67-2.50 |
| Microcrystalline cellulose | 0.63-5.13 |
| Hydroxypropyl methylcellulose | 17.23-17.80 |
| Colloidal silica | 0.91-1.0 |
| Magnesium stearate | 0.91-1.0 |
| Film coating I | 5.18-5.33 |
| Film coating II | 2.85-3.2 |

In a preferable (not limiting) embodiment of the invention, microcrystalline cellulose is microcrystalline cellulose of 101 grade.

Further, in a preferable (not limiting) embodiment of the invention, hydroxypropyl methylcellulose is hydroxypropyl methylcellulose with the molecular weight of 1,000,000 Da. Most preferably (but also without limitation), hydroxypropyl methylcellulose is hypromellose of 2208 type (K100M). A person skilled in the art will appreciate that other variations of hydroxypropyl methylcellulose having similar characteristics may be also used.

Further, in a preferable (not limiting) embodiment of the invention, colloidal silica is colloidal silica of A 200 grade. A person skilled in the art will appreciate that other variations of colloidal silica having similar characteristics may be also used.

Further, in a preferable (not limiting) embodiment of the invention, the film coating I is produced from a commercially available mixture of hydroxypropyl methylcellulose, titanium dioxide, and macrogol. In the most preferable (not limiting) embodiment of the invention, the film coating I is produced from the commercially available Opadry 03F180011 white mixture. Further, in a preferable (not limiting) embodiment of the invention, the film coating II is produced from a commercially available mixture of polyvinyl alcohol, talc, macrogol, iron oxides and titanium dioxide.

In the most preferable (not limiting) embodiment of the invention, the film coating II is produced from the commercially available Opadry II 85F 200062 purple mixture.

A person skilled in the art will appreciate that other commercially available mixtures, which are well known to those skilled in the art, may be used both for the film coating I, and for the film coating II.

**The above technical effects are also achieved by this invention owing to that a pharmaceutical composition is proposed,** which provides an increase in therapeutic activity of the quantitative and qualitative formulation of the ingredients effective in therapy of cognitive disorders of vascular origin, has neuromodulatory, antiparkinsonism, neuroprotective, and antispastic effects, slows down and inhibits glutamatergic neurotransmission and progress of neurodegenerative processes, comprises from 5 to 20 mg of memantine or its pharmaceutically acceptable salt and from 450 to 550 mg of citicoline or its pharmaceutically acceptable salt, the ratio of the quantitative and qualitative formulation of the ingredients of the pharmaceutical composition being as follows, mg:

| | |
|---|---|
| Citicoline or its pharmaceutically acceptable salt | 450-550 |
| Memantine or its pharmaceutically acceptable salt | 5-20 |
| Microcrystalline cellulose | 5-35 |
| Hydroxypropyl methylcellulose | 120.5-140.6 |
| Colloidal silica | 6.8-7.2 |
| Magnesium stearate | 6.8-7.2 |
| Film coating I | 36.4-41.5 |
| Film coating II | 21.8-24.5 |

In the most preferable (not limiting) embodiment of this invention, the pharmaceutically acceptable salt of citicoline is the citicoline monosodium salt. Further, in the most preferable (not limiting) embodiment of this invention, the memantine pharmaceutically acceptable salt is memantine hydrochloride.

In a preferable (not limiting) embodiment of the invention, microcrystalline cellulose is microcrystalline cellulose of 101 grade.

Further, in a preferable (not limiting) embodiment of the invention, hydroxypropyl methylcellulose is hydroxypropyl methylcellulose with the molecular weight of 1,000,000 Da. Most preferably (but also without limitation), hydroxypropyl methylcellulose is hypromellose of 2208 type (K100M). A person skilled in the art will appreciate that other variations of hydroxypropyl methylcellulose having similar characteristics may be used.

Further, in a preferable (not limiting) embodiment of the invention, colloidal silica is colloidal silica of A 200 grade. A person skilled in the art will appreciate that other variations of colloidal silica having similar characteristics may be used.

Further, in a preferable (not limiting) embodiment of the invention, the film coating I is produced from a commercially available mixture hydroxypropyl methylcellulose, titanium dioxide, and macrogol. In the most preferable (not limiting) embodiment of the invention, the film coating I is produced from the commercially available Opadry 03F180011 white mixture. Further, in a preferable (not limiting) embodiment of the invention, the film coating II is produced from a commercially available mixture of polyvinyl alcohol, talc, macrogol, iron oxides, and titanium dioxide.

In the most preferable (not limiting) embodiment of the invention, the film coating II is produced from the commercially available Opadry II 85F 200062 purple mixture. A person skilled in the art will appreciate that other commercially available mixtures, which are well known to those skilled in the art, may be also used both for the film coating I, and for the film coating II.

**Table 2. Examples of the quantitative and qualitative formulation of the ingredients of the ingredients of the pharmaceutical composition**

| Components | Content, % | Content, mg |
|---|---|---|
| Citicoline monosodium salt | 65.95-67.68-69.95 | 450-500-550 |
| Memantine hydrochloride | 0.67-1.30-2.50 | 5-10-20 |
| Microcrystalline cellulose | 0.63-3.95-5.13 | 5-30-35 |
| Hydroxypropyl methylcellulose | 17.23-17.66-17.80 | 120.5-133.00-140.6 |
| Colloidal silica | 0.90-0.94-1.0 | 6.8-7.0-7.2 |
| Magnesium stearate | 0.90-0.94-1.0 | 6.8-7.0-7.2 |
| Film coating I | 5.18-5.25-5.33 | 36.4-40-41.5 |
| Film coating II | 2.85-3.0-3.2 | 21.8-22-24.5 |

In a preferable (not limiting) embodiment of the invention, microcrystalline cellulose is microcrystalline cellulose of 101 grade.

Further, in a preferable (not limiting) embodiment of the invention, hydroxypropyl methylcellulose is hydroxypropyl methylcellulose having the molecular weight of 1,000,000 Da. Most preferably (but also without limitation), hydroxypropyl methylcellulose is hypromellose of 2208 type (K100M). A person skilled in the art will appreciate that other variations of hydroxypropyl methylcellulose having similar characteristics may be also used.

Further, in a preferable (not limiting) embodiment of the invention, colloidal silica is colloidal silica of A 200 grade. A person skilled in the art will appreciate that other variations of colloidal silica having similar characteristics may be also used.

Further, in a preferable (not limiting) embodiment of the invention, the film coating I is produced from a commercially available mixture of hydroxypropyl methylcellulose, titanium dioxide, and macrogol. In the most preferable (not limiting) embodiment of the invention, the film coating I is produced from the commercially available Opadry 03F180011 white mixture. Further, in a preferable (not limiting) embodiment of the invention, the film coating II is produced from a commercially available mixture of polyvinyl alcohol, talc, macrogol, iron oxides, and titanium dioxide.

In the most preferable (not limiting) embodiment of the invention, the film coating II is produced from the commercially available Opadry II 85F 200062 purple mixture. A person skilled in the art will appreciate that other commercially available mixtures, which are well known to those skilled in the art, may be also used both for the film coating I, and for the film coating II.

A technical effect is also achieved owing to that a **solid oral dosage form** based on the above pharmaceutical composition is proposed, which provides an increase in therapeutic activity of the quantitative and qualitative formulation of the ingredients, is effective in therapy of cognitive disorders of vascular origin, has neuromodulatory, antiparkinsonism, neuroprotective, and antispastic effects, slows down and inhibits glutamatergic neurotransmission and progress of neurodegenerative processes, comprises, as the active substances, from 5.00 to 20.00 mg of memantine or its pharmaceutically acceptable salt, such as memantine hydrochloride, and from 450 to 550 mg of citicoline or its pharmaceutically acceptable salt, such as the citicoline monosodium salt, the solid oral dosage form is produced in the form of a modified-release tablet coated with a film coating. The quantitative and qualitative formulation of the ingredients of this dosage form (per 1 tablet) is presented in Table 3. Not limiting (illustrative) examples of the quantitative and qualitative formulation of the ingredients of the dosage form per 1 tablet are shown in Table 4:

**Table 3. Quantitative and qualitative formulation of the ingredients of the dosage form per 1 tablet:**

| Components | Content, % | Content, mg |
|---|---|---|
| Citicoline monosodium salt | 65.95-69.95 | 450-550 |
| Memantine hydrochloride | 0.67-2.50 | 5-20 |
| Microcrystalline cellulose | 0.63-5.13 | 5-35 |
| Hydroxypropyl methylcellulose (molecular weight: 1,000,000) | 17.23-17.80 | 120.5-140.6 |
| Colloidal silica | 0.91-1.0 | 6.8-7.2 |
| Magnesium stearate | 0.91-1.0 | 6.8-7.2 |
| Film coating I | 5.18-5.33 | 36.4-41.5 |
| Film coating II | 2.85-3.2 | 21.8-24.5 |
| Total weight of a coated tablet: | | 682.3-772.0-791.0 |

**Table 4. Examples of the quantitative and qualitative formulation of the ingredients of the dosage form, per 1 tablet:**

| Components | Content, % | Content, mg |
|---|---|---|
| Citicoline monosodium salt | 65.95-67.68-69.95 | 450-500-550 |
| Memantine hydrochloride | 0.67-1.30-1.90 | 5-10-15 |
| Microcrystalline cellulose | 0.63-3.95-5.13 | 5-30-35 |
| Hydroxypropyl methylcellulose | 17.23-17.66-17.80 | 120.5-133.00-140.6 |
| Colloidal silica | 0.90-0.94-1.0 | 6.8-7.0-7.2 |
| Magnesium stearate | 0.90-0.94-1.0 | 6.8-7.0-7.2 |
| Film coating I | 5.18-5.25-5.33 | 36.4-40-41.5 |
| Film coating II | 2.85-3.0-3.2 | 21.8-22-24.5 |
| Total weight of a coated tablet: | | 682.3-772.0-791.0 |

In a preferable (not limiting) embodiment of the invention, microcrystalline cellulose is microcrystalline cellulose of 101 grade.

Further, in a preferable (not limiting) embodiment of the invention, hydroxypropyl methylcellulose is hydroxypropyl methylcellulose having the molecular weight of 1,000,000 Da. Most preferably (but also without limitation), hydroxypropyl methylcellulose is hypromellose of 2208 type (K100M). A person skilled in the art will appreciate that other variations of hydroxypropyl methylcellulose having similar characteristics may be also used.

Further, in a preferable (not limiting) embodiment of the invention, colloidal silica is colloidal silica of A 200 grade. A person skilled in the art will appreciate that other variations of colloidal silica having similar characteristics may be also used.

Further, in a preferable (not limiting) embodiment of the invention, the film coating I is produced from a commercially available mixture of hydroxypropyl methylcellulose, titanium dioxide, and macrogol. In the most preferable (not limiting) embodiment of the invention, the film coating I is produced from the commercially available Opadry 03F180011 white mixture. Further, in a preferable (not limiting) embodiment of the invention, the film coating II is produced from a commercially available mixture of polyvinyl alcohol, talc, macrogol, iron oxides, and titanium dioxide.

In the most preferable (not limiting) embodiment of the invention, the film coating II is produced from the commercially available Opadry II 85F 200062 purple mixture. A person skilled in the art will appreciate that other commercially available mixtures, which are well known to those skilled in the art, may be used both for the film coating I, and for the film coating II.

The technical effects are further achieved by this invention owing to that a **solid oral dosage form** is proposed, which provides an increase in therapeutic activity of the quantitative and qualitative formulation of the ingredients, is effective in therapy of cognitive disorders of vascular origin, has neuromodulatory, antiparkinsonism, neuroprotective, and antispastic effects, slows down and inhibits glutamatergic neurotransmission and progress of neurodegenerative processes, comprises, as the active substances, memantine or its pharmaceutically acceptable salt in the range from 5,00 to 20,00 mg and citicoline or its pharmaceutically acceptable salt in the range from 450 to 550 mg, this solid oral dosage form being produced in the form of a modified-release tablet coated with a film coating and contains the following quantitative and qualitative formulation of the ingredients per 1 tablet, wt%:

| | |
|---|---|
| Citicoline or its pharmaceutically acceptable salt | 65.95-69.95 |
| Memantine or its pharmaceutically acceptable salt | 0.67-2.50 |
| Microcrystalline cellulose | 0.63-5.13 |
| Hydroxypropyl methylcellulose | 17.23-17.80 |
| Colloidal silica | 0.91-1.0 |
| Magnesium stearate | 0.91-1.0 |
| Film coating I | 5.18-5.33 |
| Film coating II | 2.85-3.2 |

In the most preferable (not limiting) embodiment of this invention, the citicoline pharmaceutically acceptable salt is the citicoline monosodium salt, and the memantine pharmaceutically acceptable salt is memantine hydrochloride.

Further, in a preferable (not limiting) embodiment of the invention, microcrystalline cellulose is microcrystalline cellulose of 101 grade.

Further, in a preferable (not limiting) embodiment of the invention, hydroxypropyl methylcellulose is hydroxypropyl methylcellulose having the molecular weight of 1,000,000 Da. Most preferably (but also without limitation), hydroxypropyl methylcellulose is hypromellose of 2208 type (K100M). A person skilled in the art will appreciate that other variations of hydroxypropyl methylcellulose having similar characteristics may be also used.

Further, in a preferable (not limiting) embodiment of the invention, colloidal silica is colloidal silica of A 200 grade. A person skilled in the art will appreciate that other variations of colloidal silica having similar characteristics may be also used.

Further, in a preferable (not limiting) embodiment of the invention, the film coating I is produced from a commercially available mixture of hydroxypropyl methylcellulose, titanium dioxide, and macrogol. In the most preferable (not limiting) embodiment of the invention, the film coating I is produced from the commercially available Opadry 03F180011 white mixture. Further, in a preferable (not limiting) embodiment of the invention, the film coating II is produced from a commercially available mixture of polyvinyl alcohol, talc, macrogol, iron oxides, and titanium dioxide.

In the most preferable (not limiting) embodiment of the invention, the film coating II is produced from the commercially available Opadry II 85F 200062 purple mixture. A person skilled in the art will appreciate that other commercially available mixtures, which are well known to those skilled in the art, may be also used both for the film coating I, and for the film coating II.

**The technical effects are also achieved by this invention owing to that a solid oral dosage form** is proposed, which provides an increase in therapeutic activity of the quantitative and qualitative formulation of the ingredients, is effective in therapy of cognitive disorders of vascular origin, has neuromodulatory, antiparkinsonism, neuroprotective, and antispastic effects, slows down and inhibits glutamatergic neurotransmission and progress of neurodegenerative processes, comprises, as the active substances, memantine or its pharmaceutically acceptable salt, e.g., memantine hydrochloride, in the range from 5,00 to 20,00 mg and citicoline or its pharmaceutically acceptable salt in the range from 450 to 550 mg, is produced in the form of a modified-release tablet coated with a film coating, the quantitative and qualitative formulation of the ingredients per 1 tablet being, mg:

| | |
|---|---|
| Citicoline or its pharmaceutically acceptable salt | 450-550 |
| Memantine or its pharmaceutically acceptable salt | 5-20 |
| Microcrystalline cellulose | 5-35 |
| Hydroxypropyl methylcellulose | 120.5-140.6 |
| Colloidal silica | 6.8-7.2 |
| Magnesium stearate | 6.8-7.2 |
| Film coating I | 36.4-41.5 |
| Film coating II | 21.8-24.5 |

In the most preferable (not limiting) embodiment of this invention, the citicoline pharmaceutically acceptable salt is the citicoline monosodium salt, and the memantine pharmaceutically acceptable salt is memantine hydrochloride.

Further, in a preferable (not limiting) embodiment of the invention, microcrystalline cellulose is microcrystalline cellulose of 101 grade.

Further, in a preferable (not limiting) embodiment of the invention, hydroxypropyl methylcellulose is hydroxypropyl methylcellulose having the molecular weight of 1,000,000 Da. Most preferably (but also without limitation), hydroxypropyl methylcellulose is hypromellose of 2208 type (K100M). A person skilled in the art will appreciate that other variations of hydroxypropyl methylcellulose having similar characteristics may be also used.

Further, in a preferable (not limiting) embodiment of the invention, colloidal silica is colloidal silica of A 200 grade. A person skilled in the art will appreciate that other variations of colloidal silica having similar characteristics may be also used.

Further, in a preferable (not limiting) embodiment of the invention, the film coating I is produced from a commercially available mixture of hydroxypropyl methylcellulose, titanium dioxide, and macrogol. In the most preferable (not limiting) embodiment of the invention, the film coating I is produced from the commercially available Opadry 03F180011 white mixture. Further, in a preferable (not limiting) embodiment of the invention, the film coating II is produced from a commercially available mixture of polyvinyl alcohol, talc, macrogol, iron oxides, and titanium dioxide.

In the most preferable (not limiting) embodiment of the invention, the film coating II is produced from the commercially available Opadry II 85F 200062 purple mixture. A person skilled in the art will appreciate that other commercially available mixtures, which are well known to those skilled in the art, may be also used both for the film coating I, and for the film coating II.

**Table 4. Examples of the quantitative and qualitative formulation of the ingredients of the dosage form per 1 tablet:**

| Components | Content, % | Content, mg |
|---|---|---|
| Citicoline monosodium salt | 65.95-67.68-69.95 | 450-500-550 |
| Memantine hydrochloride | 0.67-1.30-1.90 | 5-10-15 |
| Microcrystalline cellulose | 0.63-3.95-5.13 | 5-30-35 |
| Hydroxypropyl methylcellulose | 17.23-17.66-17.80 | 120.5-133.00-140.6 |
| Colloidal silica | 0.90-0.94-1.0 | 6.8-7.0-7.2 |
| Magnesium stearate | 0.90-0.94-1.0 | 6.8-7.0-7.2 |
| Film coating I | 5.18-5.25-5.33 | 36.4-40-41.5 |
| Film coating II | 2.85-3.0-3.2 | 21.8-22-24.5 |
| Total weight of a coated tablet: | | 682.3-772.0-791.0 |

In a preferable (not limiting) embodiment of the invention, microcrystalline cellulose is microcrystalline cellulose of 101 grade.

Further, in a preferable (not limiting) embodiment of the invention, hydroxypropyl methylcellulose is hydroxypropyl methylcellulose having the molecular weight of 1,000,000 Da. Most preferably (but also without limitation), hydroxypropyl methylcellulose is hypromellose of 2208 type (K100M). A person skilled in the art will appreciate that other variations of hydroxypropyl methylcellulose having similar characteristics may be also used.

Further, in a preferable (not limiting) embodiment of the invention, colloidal silica is colloidal silica of A 200 grade. A person skilled in the art will appreciate other variations of colloidal silica having similar characteristics may be also used.

Further, in a preferable (not limiting) embodiment of the invention, the film coating I is produced from a commercially available mixture of hydroxypropyl methylcellulose, titanium dioxide, and macrogol. In the most preferable (not limiting) embodiment of the invention, the film coating I is produced from the commercially available Opadry 03F180011 white mixture. Further, in a preferable (not limiting) embodiment of the invention, the film coating II is produced from a commercially available mixture of polyvinyl alcohol, talc, macrogol, iron oxides, and titanium dioxide.

In the most preferable (not limiting) embodiment of the invention, the film coating II is produced from the commercially available Opadry II 85F 200062 purple mixture. A person skilled in the art will appreciate that other commercially available mixtures, which are well known to those skilled in the art, may be also used both for the film coating I, and for the film coating II.

The technical effect relating to simplification and cost reduction of the dosage form and the pharmaceutical composition, which are intended for therapy of cognitive disorders of vascular origin, have neuromodulatory, antiparkinsonism, neuroprotective, and antispastic effects, slow down and inhibit glutamatergic neurotransmission and progress of neurodegenerative processes, is achieved by this invention owing to that a **method for producing a dosage form based on the pharmaceutical composition** is proposed, wherein magnesium stearate, the citicoline monosodium salt - memantine hydrochloride, microcrystalline cellulose, including that of 101 grade, hydroxypropyl methylcellulose, colloidal silica, including that of A 200 grade, are sifted through a sieve with a mesh diameter of not more than 0.5 mm; then the citicoline monosodium salt, microcrystalline cellulose and hydroxypropyl methylcellulose are loaded into a mixer-granulator or a similar mixing device, mixed to a homogenous state at a mixing rate of 160-240 rpm and for a mixing period from 2 to 10 minutes; then the mixture is moistened with purified water at constant stirring for not more than 5 minutes, a stirring rate being from 150 to 400 rpm; after that, the obtained granulated material is dried in the fluidized bed conditions, while the following parameters being maintained: an input air temperature - 45-65°C, residual moisture of the granulated material - from 2.5 to 4.5%; then, the obtained mixture is dusted with colloidal silica: after that, the obtained mixture is pressed in a rotary tableting press with the use of a tablet tooling having a diameter from 10 to 15 mm, while the following parameters of the tableting process being monitored: hardness - 150-250 H, grateness - not more than 1.0%, height - not more than 7.0 mm; after that, a film coating is applied to the obtained tablet cores; in the process of dissolving a film coating mixture of hydroxypropyl methylcellulose, titanium dioxide and macrogol, a pharmaceutical substance of memantine hydrochloride is added in portions and is mixed until formation of a homogenous suspension, after that, the obtained coating is applied onto tablet cores at 35-40°C with intermittent mixing, including that in the drum of a coater; then, a film coating solution is prepared from a mixture of polyvinyl alcohol, talc, macrogol, iron oxides, and titanium dioxide; upon preparation of a solution of film-forming material, it is applied to the tablets in the same conditions as are used for the previous coating. The preferable quantitative and qualitative formulation of theingridients of the dosage form per 1 tablet is presented in Table 3. Preferable (not limiting) examples of the quantitative and qualitative formulation of the ingredients of the dosage form per 1 tablet are shown in Table 4.

The technical effect relating to simplification and cost reduction of the dosage form and the pharmaceutical composition, which are intended for therapy of cognitive disorders of vascular origin, have neuromodulatory, antiparkinsonism, neuroprotective, and antispastic effects, slow down and inhibit glutamatergic neurotransmission and progress of neurodegenerative processes, is achieved by this invention owing to that a **method is proposed for producing a dosage form based on the pharmaceutical composition** providing an increase in therapeutic activity of the quantitative and qualitative formulation of the ingredients, effective in therapy of cognitive disorders of vascular origin and having neuromodulatory, antiparkinsonism, neuroprotective, and antispastic effects, slowing down and inhibiting glutamatergic neurotransmission and progress of neurodegenerative processes; in accordance with this method, magnesium stearate, citicoline or its pharmaceutically acceptable salt, memantine or its pharmaceutically acceptable salt, microcrystalline cellulose, hydroxypropyl methylcellulose, and colloidal silica are sifted through a sieve with a mesh diameter of not more than 0.5 mm; then, citicoline or its pharmaceutically acceptable salt, microcrystalline cellulose and hydroxypropyl methylcellulose are loaded into a mixer-granulator or a similar mixing device, mixed to a homogenous state at a mixing rate of 160-240 rpm and for a mixing period from 2 to 10 minutes; then, the mixture is moistened with purified water at constant stirring, a stirring rate being within 150-400 rpm, and a stirring time should be not more than 5 minutes; after that, the obtained granulated material is dried in the fluidized bed conditions, while the following parameters being maintained: an input air temperature - 45-65°C, residual moisture of the granulated material - from 2.5 to 4.5%; then, the obtained mixture is dusted with colloidal silica; after that, the obtained mixture is pressed in a rotary tableting press with the use of a tablet tooling having a diameter from 10 to 15 mm, while the following parameters of the tableting process being monitored: hardness - 150-250 H, grateness - not more than 1.0%, height - not more than 7.0 mm; after that, a film coating is applied to the obtained tablet cores; in the process of dissolving a film coating mixture of hydroxypropyl methylcellulose, titanium dioxide and macrogol, a pharmaceutical substance of memantine hydrochloride is added in portions and is mixed until formation of a homogenous suspension; after that, the obtained coating is applied onto tablet cores at 35-40°C with intermittent mixing, including that in the drum of a coater; then, a solution of a film-forming material for a film coating II is prepared from a mixture of polyvinyl alcohol, talc, macrogol, iron oxides, and titanium dioxide; upon preparation of the solution of the film coating II, it is applied to the tablets in the same conditions as are used for the previous coating, following quantitative and qualitative formulation of the ingredients of the dosage form per 1 tablet being used, wt%:

| | |
|---|---|
| Citicoline or its pharmaceutically acceptable salt | 65.95-69.95 |
| Memantine or its pharmaceutically acceptable salt | 0.67-2.50 |
| Microcrystalline cellulose | 0.63-5.13 |
| Hydroxypropyl methylcellulose | 17.23-17.80 |
| Colloidal silica | 0.91-1.0 |
| Magnesium stearate | 0.91-1.0 |
| Film coating I | 5.18-5.33 |
| Film coating II | 2.85-3.2 |

In the most preferable (not limiting) embodiment of this invention, the citicoline pharmaceutically acceptable salt is the citicoline monosodium salt, the memantine pharmaceutically acceptable salt is memantine hydrochloride.

In a preferable (not limiting) embodiment of the method according to the invention, microcrystalline cellulose is microcrystalline cellulose of 101 grade.

Further, in a preferable (not limiting) embodiment of the method according to the invention, hydroxypropyl methylcellulose is hydroxypropyl methylcellulose having the molecular weight of 1,000,000 Da. Most preferably (but also without limitation), hydroxypropyl methylcellulose is hypromellose of 2208 type (K100M). A person skilled in the art will appreciate that other variations of hydroxypropyl methylcellulose having similar characteristics may be also used.

Further, in a preferable (not limiting) embodiment of the method according to the invention, colloidal silica is colloidal silica of A 200 grade. A person skilled in the art will appreciate that other variations of colloidal silica having similar characteristics may be also used.

Further, in a preferable (not limiting) embodiment of the method according to the invention, the film coating I is produced from a commercially available mixture of hydroxypropyl methylcellulose, titanium dioxide, and macrogol. In the most preferable (not limiting) embodiment of the invention, the film coating I is produced from the commercially available Opadry 03F180011 white mixture.

Further, in a preferable (not limiting) embodiment of the method according to the invention, the film coating II is produced from a commercially available mixture of polyvinyl alcohol, talc, macrogol, iron oxides, and titanium dioxide.

In the most preferable (not limiting) embodiment of the invention, the film coating II is produced from a commercially available Opadry II 85F 200062 purple mixture. A person skilled in the art will appreciate that other commercially available mixtures, which are well known to those skilled in the art, may be also used both for the film coating I, and for the film coating II.

The technical effect relating to simplification and cost reduction of the dosage form and the pharmaceutical composition, which are intended for therapy of cognitive disorders of vascular origin, have neuromodulatory, antiparkinsonism, neuroprotective, and antispastic effects, slow down and inhibit glutamatergic neurotransmission and progress of neurodegenerative processes, is also achieved by this invention owing to that the **method is proposed in the invention for producing a dosage form based on the pharmaceutical composition** providing an increase in therapeutic activity of the quantitative and qualitative formulation of the ingredients, effective in therapy of cognitive disorders of vascular origin and having neuromodulatory, antiparkinsonism, neuroprotective, and antispastic effects, slowing down and inhibiting glutamatergic neurotransmission and progress of neurodegenerative processes, which includes from 5 to 20 mg of memantine or its pharmaceutically acceptable salt, e.g., memantine hydrochloride, and from 450 to 550 mg of citicoline or its pharmaceutically acceptable salt, e.g., citicoline monosodium; wherein, according to this method, magnesium stearate, citicoline or its pharmaceutically acceptable salt, memantine or its pharmaceutically acceptable salt, microcrystalline cellulose, hydroxypropyl methylcellulose and colloidal silica are sifted through a sieve with a mesh diameter not more than 0.5 mm; then, the citicoline monosodium salt, microcrystalline cellulose and hydroxypropyl methylcellulose are loaded into a mixer-granulator or a similar mixing device, mixed to a homogenous state at a mixing rate of 160-240 rpm and for a mixing time from 2 to 10 minutes; after that, the mixture is moistened with purified water at constant stirring, while a stirring rate of 150-400 rpm being maintained, for not more than 5 minutes; then, the obtained granulated material is dried in the fluidized bed conditions, while the following parameters being maintained: an input air temperature - 45-65°C, residual moisture of the granulated material - from 2.5 to 4.5%; the obtained granulated material is sifted through a sieve with a mesh diameter not more than 1.0 mm; after that, the obtained mixture is dusted with colloidal silica for 3-5 minutes; then, a portion of hydroxypropyl methylcellulose is added to the mixture, stirring is continued for another 8-10 minutes; after that, magnesium stearate is added, and stirring is continued for another 2-3 minutes. The obtained tableting mixture is pressed in a rotary tableting press with the use of a tablet tooling having a diameter from 10 to 15 mm; the following parameters of the tableting process are monitored: hardness - 150-290 H, grateness - not more than 1.0%, height - not more than 7.0 mm; after that, a film coating is applied to the obtained tablet cores; in the process of dissolving a film coating mixture of hydroxypropyl methylcellulose, titanium dioxide, and macrogol, a pharmaceutical substance of memantine hydrochloride is added in portions and is mixed until formation of a homogenous suspension; after that, the obtained coating is applied onto the tablet cores at 35-40°C with intermittent mixing, including that in the drum of a coater; then, a solution of a film coating is prepared from a mixture of polyvinyl alcohol, talc, macrogol, iron oxides, and titanium dioxide; upon preparation of a solution of the film-forming material, it is applied to the tablets in the same conditions as are used for the previous coating, the quantitative and qualitative formulation of the ingredients of the dosage form being, per 1 tablet, mg:

| | |
|---|---|
| Citicoline or its pharmaceutically acceptable salt | 450-550 |
| Memantine or its pharmaceutically acceptable salt | 5-20 |
| Microcrystalline cellulose | 5-35 |
| Hydroxypropyl methylcellulose | 120.5-140.6 |
| Colloidal silica | 6.8-7.2 |
| Magnesium stearate | 6.8-7.2 |
| Film coating I | 36.4-41.5 |
| Film coating II | 21.8-24.5 |

In the most preferable (not limiting) embodiment of this invention, a citicoline pharmaceutically acceptable salt is the citicoline monosodium salt, a memantine pharmaceutically acceptable salt is memantine hydrochloride.

In a preferable (not limiting) embodiment of the method according to the invention, microcrystalline cellulose is microcrystalline cellulose of 101 grade.

Further, in a preferable (not limiting) embodiment of the method according to the invention, hydroxypropyl methylcellulose is hydroxypropyl methylcellulose having the molecular weight of 1,000,000 Da. Most preferably (but also without limitation), hydroxypropyl methylcellulose is hypromellose of 2208 type (K100M). A person skilled in the art will appreciate that other variations of hydroxypropyl methylcellulose having similar characteristics may be also used.

Further, in a preferable (not limiting) embodiment of the method according to the invention, colloidal silica is colloidal silica of A 200 grade. A person skilled in the art will appreciate that other variations of colloidal silica having similar characteristics may be also used.

Further, in a preferable (not limiting) embodiment of the method according to the invention, the film coating I is produced from a commercially available mixture of hydroxypropyl methylcellulose, titanium dioxide, and macrogol. In the most preferable (not limiting) embodiment of the invention, the film coating I is produced from the commercially available Opadry 03F180011 white mixture.

Further, in a preferable (not limiting) embodiment of the method according to the invention, the film coating II is produced from a commercially available mixture of polyvinyl alcohol, talc, macrogol, iron oxides, and titanium dioxide.

In the most preferable (not limiting) embodiment of the invention, the film coating II is produced from the commercially available Opadry II 85F 200062 purple mixture. A person skilled in the art will appreciate that other commercially available mixtures, which are well known to those skilled in the art, may be also used both for the film coating I, and for the film coating II.

Furthermore, the technical effects are achieved by this invention also owing to that a **drug product** is proposed **in the form of a modified-release tablet,** comprising citicoline or its pharmaceutically acceptable salt and memantine or its pharmaceutically acceptable salt as the pharmaceutical substances, microcrystalline cellulose as the excipient, hypromellose as the matrix agent, colloidal silica as the glidant, magnesium stearate as the lubricant, a film coating as the isolating coating (film coating I), the protective coating (film coating II) and the carrier (film coating Ia), and having the following quantitative and qualitative formulation of the ingredients of the dosage form, per 1 tablet, wt%:

| | |
|---|---|
| Citicoline pharmaceutically acceptable salt (equivalent to Citicoline base) | 69.95 (66.93) |
| Microcrystalline cellulose | 4.08 |
| Hypromellose | 17.80 |
| Colloidal silica | 0.94 |
| Magnesium stearate | 0.94 |
| Film coating I | 1.34 |
| Memantine pharmaceutically acceptable salt | 0.67 |
| Film coating Ia | 2.68 |
| Film coating II | 1.61 |

In the most preferable (not limiting) embodiment of this invention, the citicoline pharmaceutically acceptable salt is the citicoline monosodium salt, the memantine pharmaceutically acceptable salt is memantine hydrochloride.

Further, in a preferable (not limiting) embodiment of the drug product (modified-release tablet) according to this invention, microcrystalline cellulose is microcrystalline cellulose of 101 grade.

Further, in a preferable (not limiting) embodiment of the drug product (modified-release tablet) according to this invention, hydroxypropyl methylcellulose is hydroxypropyl methylcellulose having the molecular weight of 1,000,000 Da. Most preferably (but also without limitation), hydroxypropyl methylcellulose is hypromellose of 2208 type (K100M). A person skilled in the art will appreciate that other variations of hydroxypropyl methylcellulose having similar characteristics may be also used.

Further, in a preferable (not limiting) embodiment of the drug product (modified-release tablet) according to this invention, colloidal silica is colloidal silica of A 200 grade. A person skilled in the art will appreciate that other variations of colloidal silica having similar characteristics may be also used.

Further, in a preferable (not limiting) embodiment of the drug product (modified-release tablet) according to this invention, a film coating I is produced from a commercially available mixture of hydroxypropyl methylcellulose, titanium dioxide, and macrogol. In the most preferable (not limiting) embodiment of the invention, the film coating I is produced from the commercially available Opadry 03F180011 white mixture.

Further, in a preferable (not limiting) embodiment of the drug product (modified-release tablet) according to this invention, a film coating II is produced from a commercially available mixture of polyvinyl alcohol, talc, macrogol, iron oxides, and titanium dioxide.

In the most preferable (not limiting) embodiment of the invention, the film coating II is produced from the commercially available Opadry II 85F 200062 purple mixture. A person skilled in the art will appreciate that other commercially available mixtures, which are well known to those skilled in the art, may be also used both for the film coating I, and for the film coating II.

Furthermore, the technical effects are also achieved by this invention owing to that the **drug product** is proposed **in the form of a modified-release tablet,** comprising citicoline or its pharmaceutically acceptable salt, e.g. citicoline monosodium, and memantine or its pharmaceutically acceptable salt, e.g. memantine hydrochloride, as the pharmaceutical substances, microcrystalline cellulose as the excipient, hypromellose as the matrix agent, colloidal silica as the glidant, magnesium stearate as the lubricant, a film coating as the coating and carrier, and having the following quantitative and qualitative formulation of the ingridients of the dosage form, per 1 tablet, mg:

| | |
|---|---|
| Citicoline pharmaceutically acceptable salt (equivalent to Citicoline base) | 522.50 (500.00) |
| Microcrystalline cellulose | 30.50 |
| Hypromellose | 133.00 |
| Colloidal silica | 7.00 |
| Magnesium stearate | 7.00 |
| Film coating I | 10.00 |
| Memantine pharmaceutically acceptable salt | 5.00 |
| Film coating Ia | 20.00 |
| Film coating II | 12.00 |

In the most preferable (not limiting) embodiment of this invention, the citicoline pharmaceutically acceptable salt is the citicoline monosodium salt, the memantine pharmaceutically acceptable salt is memantine hydrochloride.

In a preferable (not limiting) embodiment of the drug product (modified-release tablet) according to this invention, microcrystalline cellulose is microcrystalline cellulose of 101 grade.

Further, in a preferable (not limiting) embodiment of the drug product (modified-release tablet) according to this invention, hydroxypropyl methylcellulose is hydroxypropyl methylcellulose having the molecular weight of 1,000,000 Da. Most preferably (but also without limitation), hydroxypropyl methylcellulose is hypromellose of 2208 type (K100M). A person skilled in the art will appreciate that other variations of hydroxypropyl methylcellulose having similar characteristics may be also used.

Further, in a preferable (not limiting) embodiment of the drug product (modified-release tablet) according to this invention, colloidal silica is colloidal silica of A 200 grade. A person skilled in the art will appreciate that other variations of colloidal silica having similar characteristics may be also used.

Further, in a preferable (not limiting) embodiment of the drug product (modified-release tablet) according to this invention, the film coating I is produced from a commercially available mixture of hydroxypropyl methylcellulose, titanium dioxide, and macrogol. In the most preferable (not limiting) embodiment of the invention, the film coating I is produced from the commercially available Opadry 03F180011 white mixture.

Further, in a preferable (not limiting) embodiment of the drug product (modified-release tablet) according to this invention, the film coating II is produced from a commercially available mixture of polyvinyl alcohol, talc, macrogol, iron oxides, and titanium dioxide.

In the most preferable (not limiting) embodiment of the invention, the film coating II is produced from the commercially available Opadry II 85F 200062 purple mixture. A person skilled in the art will appreciate that other commercially available mixtures, which are well known to those skilled in the art, may be also used both for the film coating I, and for the film coating II.

**Table 5. Examples of the quantitative and qualitative formulation of the ingredients, per 1 tablet, comprising memantine hydrochloride (equivalent to 5 mg of the memantine base) and citicoline sodium (equivalent to 500 mg of the citicoline base) as the active substances.**

| **Components** | **Content, %** | **Content, mg/tablet** | **Purpose** |
|---|---|---|---|
| Citicoline sodium (equivalent to citicoline base) | 69.95 | 522.50 (500.00) | Pharmaceutical substance |
| Microcrystalline cellulose 101 | 4.08 | 30.50 | Excipient |
| Hypromellose of 2208 type (K100M) | 17.80 | 133.00 | Matrix agent |
| Colloidal silica A 200 | 0.94 | 7.00 | Glidant |
| Magnesium stearate | 0.94 | 7.00 | Lubricant |
| **Total: tablet core weight** | | **700.00** | - |
| Film coating Opadry 03F180011 white (hypromellose, titanium dioxide, macrogol) | 1.34 | 10.00 | Coating |
| **Total: coated tablet weight** | | **710.00** | **-** |
| Memantine hydrochloride | 0.67 | 5.00 | Pharmaceutical substance |
| Film coating I (Opadry 03F180011 white) | 2.68 | 20.00 | Coating/Carrier |
| **Total: coated tablet weight** | | **735.00** | - |
| Film coating II (Opadry II 85F200062 Purple) | 1.61 | 12.00 | Coating |
| **Total: coated tablet weight** | | 747.00 | - |

Furthermore, the technical effects are also achieved by this invention owing to that the **drug product** is proposed **in the form of a modified-release tablet,** comprising citicoline or its pharmaceutically acceptable salt, e.g. citicoline monosodium, and memantine or its pharmaceutically acceptable salt, e.g. memantine hydrochloride, as the pharmaceutical substances, microcrystalline cellulose as the excipient, hypromellose as the matrix agent, colloidal silica as the glidant, magnesium stearate as the lubricant, a film coating as the coating and carrier, and having the following quantitative and qualitative formulation of the ingredients of the dosage form, per 1 tablet, wt%:

| | |
|---|---|
| Citicoline sodium | 67.68 |
| (equivalent to citicoline base) | (64.77) |
| Microcrystalline cellulose 101 | 3.95 |
| Hypromellose of 2208 type (K100M) | 17.23 |
| Colloidal silica A 200 | 0.91 |
| Magnesium stearate | 0.91 |
| Film coating Ia (Opadry 03F180011 white) | 1.30 |
| Memantine hydrochloride | 1.30 |
| Film coating Ib (Opadry 03F180011 white) | 5.18 |
| Film coating II (Opadry II 85F200062 Purple) | 1.55 |

Furthermore, the technical effects are also achieved by this invention owing to that the **drug product** is proposed **in the form of a modified-release tablet,** comprising citicoline or its pharmaceutically acceptable salt, e.g. citicoline sodium, and memantine or its pharmaceutically acceptable salt, e.g. memantine hydrochloride, as the active substances, microcrystalline cellulose as the excipient, hypromellose as the matrix agent, colloidal silica as the glidant, magnesium stearate as the lubricant, a film coating as the coating and carrier, and having the following quantitative and qualitative formulation of the ingredients of the dosage form, per 1 tablet, mg:

| | |
|---|---|
| Citicoline sodium | 522.50 |
| (equivalent to citicoline base) | (500.00) |
| Microcrystalline cellulose 101 | 30.50 |
| Hypromellose of 2208 type (K100M) | 133.00 |
| Colloidal silica A 200 | 7.00 |
| Magnesium stearate | 7.00 |
| Film coating Opadry 03F180011 white | 10.00 |
| (hypromellose, titanium dioxide, macrogol) | |
| Memantine hydrochloride | 10.00 |
| Film coating Opadry 03F180011 white | 40.00 |
| (hypromellose, titanium dioxide, macrogol) | |
| Film coating Opadry II 85F200062 purple (polyvinyl alcohol, titanium dioxide, macrogol, talc, iron oxide red, iron oxide black) | 12.00 |

**Table 6. Examples of the quantitative and qualitative formulation of the ingredients per 1 tablet comprising memantine in the quantity of 10 mg and citicoline in the quantity of 500 mg as the active substances.**

| **Components** | **Content, %** | **Content, mg/tablet** | **Purpose** | |
|---|---|---|---|---|
| Citicoline sodium (equivalent to citicoline base) | 67.68 | 522.50 (500.00) | Pharmaceutical substance | |
| Microcrystalline cellulose 101 | | 3.95 | 30.50 | Excipient |
| Hypromellose of 2208 type (K100M) | | 17.23 | 133.00 | Matrix agent |
| Colloidal silica A 200 | | 0.91 | 7.00 | Glidant |
| Magnesium stearate | | 0.91 | 7.00 | Lubricant |
| **Total: tablet core weight** | | | **700.00** | **-** |
| Film coating Opadry 03F180011 white (hypromellose, titanium dioxide, macrogol) | 1.30 | | 10.00 | Coating |
| **Total: coated tablet weight** | | | **710.00** | - |
| Memantine hydrochloride | 1.30 | | 10.00 | Pharmaceutical substance |
| Film coating Opadry 03F180011 white (hypromellose, titanium dioxide, macrogol) | 5.18 | | 40.00 | Coating/Carrier |
| **Total: coated tablet weight** | | | **760.00** | **-** |
| Film coating Opadry II 85F200062 Purple (polyvinyl alcohol, titanium dioxide, macrogol, talc, iron oxide red, iron oxide black) | 1.55 | | 12.00 | Coating |
| **Total: coated tablet weight** | | | **772.00** | - |

Furthermore, the technical effects are also achieved by this invention owing to that the method is proposed for producing of the **drug product in the form of a modified-release tablet, which comprises,** as the active substances, citicoline sodium and memantine hydrochloride, and, further, microcrystalline cellulose as the excipient, hypromellose as the matrix agent, colloidal silica as the glidant, magnesium stearate as the lubricant, a film coating as the coating and carrier, and has the following quantitative and qualitative formulation of the ingredients of the dosage form, per 1 tablet, mg:

| | |
|---|---|
| Citicoline sodium | 522.50 |
| (equivalent to citicoline base) | (500.00) |
| Microcrystalline cellulose 101 | 30.50 |
| Hypromellose of 2208 type (K100M) | 133.00 |
| Colloidal silica A 200 | 7.00 |
| Magnesium stearate | 7.00 |
| Film coating Opadry 03F180011 white | 10.00 |
| (hypromellose, titanium dioxide, macrogol) | |
| Memantine hydrochloride | 5.00-10.00 |
| Film coating Opadry 03F180011 white (hypromellose, titanium dioxide, macrogol) | 20.00-40.00 |
| Film coating Opadry II 85F200062 | 12.00 |
| Purple (polyvinyl alcohol, titanium dioxide, macrogol, talc, iron oxide red, iron oxide black) | |

**and is characterized in that the above method comprises:**
**sifting of raw materials, wherein magnesium stearate** is sifted manually through a 0.5 mm sieve, residues are rubbed through the sieve manually; then, citicoline sodium is sifted on a sifting apparatus, e.g., on CISA RP 200 N Vibrosieve, through a 0.50 sieve, and memantine hydrochloride is sifted, e.g., on CISA RP 200 N Vibrosieve through a 0.075 mm sieve; microcrystalline cellulose 101, hypromellose K100M, and colloidal silica are sifted, e.g., on CISA RP 200 N Vibrosieve through a 0.5 mm sieve;
**weighing of raw materials, wherein** a raw material of each kind is weighed into individual clean containers, marked and provided to the next steps;
**a process of preparing a moistener solution, wherein** purified water is used as the moistener solution, the amount of purified water being calculated depending on a dry mixture weight and the mixture/water ratio being 3.4:1;
**a process of preparing a tableting mixture, wherein** sifted citicoline sodium, sifted microcrystalline cellulose, e.g. of 101 grade, and hypromellose, including that of 2208 type, are loaded into a mixer-granulator, and then the components are mixed to a homogenous state while being monitored visually, the mixing parameters depending on a laboratory batch volume.

In particular,
Parameters for a laboratory batch of 500 tablets:
Bowl volume - 3.0 L
Mixing rate - 200.0 rpm
Mixing period - 120 s;
**next, a granulation process is conducted, wherein,** while continuously stirring the mixture in the mixer-granulator, the moistener solution is added, stirring is continued until formation of a fine and loose granulated material (monitored visually), the granulation parameters depending on a laboratory batch volume, including
Parameters for a laboratory batch of 500 tablets:
Stirring rate - 200.0-350.0 rpm.
Stirring period - 60 s;
**further, the granulated material is dried, wherein** the granulated material is dried in a drying cabinet (a fluidized-bed drier), the drying process parameters depending on equipment used and a laboratory batch volume, including
Parameters for a laboratory batch of 500 tablets:

| | |
|---|---|
| Input air temperature | 50-65°C. |
| Drying period | 3-6 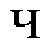 |
| Residual moisture of granulated material | from 2.5 to 4.5%; |

**after that, the granulated material is calibrated, i.e.,** the granulated material is sifted, e.g., on CISA RP 200 N Vibrosieve through a 1.0 mm sieve (0.710 mm), and remains are rubbed through the 1.0 sieve (0.710 mm) manually;
**further, dusting is performed, wherein** the granulated material and sifted colloidal silica are loaded into a mixer and mixed to a homogenous state (while monitoring visually), the mixing parameters depending on an equipment type and a laboratory batch volume, including

Parameters for a laboratory batch of 500 tablets:
Bowl volume - 3.0 L
Mixing period - 300 s,
meanwhile, if agglomerates of colloidal silica are present, it is allowed to conduct sifting through a 1.0 mm sieve, agglomerates of colloidal silica are rubbed through the sieve manually; hypromellose, including that of 2208 type, is added to the mixer, and the mixing is continued until a homogenous state is reached (while monitoring visually), the mixing parameters depending on an equipment type and a laboratory batch volume, including
Parameters for a laboratory batch of 500 tablets:
Bowl volume - 3.0 L
Mixing period - 600 s,
sifted magnesium stearate is added to the mixer, and the components are mixed to a homogenous state (while monitoring visually), the mixing parameters depending on an equipment type and a laboratory batch volume, including
Parameters for a laboratory batch of 500 tablets:
Bowl volume - 3.0 L
Mixing period - from 120 to 180 s,

the obtained tableting mixture is discharged into a marked container, a representative sample is taken for checking according to the existing specification; then, a **tableting** process **is performed** in a tableting press, e.g. (but without limitation) Futorque X-1; the tableting process being conducted on a round 12.00 mm tooling; in the beginning of the process, a tableting press is adjusted for producing average-weight tablets, including 700.0 mg;
after an average weight of tablets is adjusted, the other parameters of the tableting press are adjusted, such as:
   hardness - 200-290 H;
   grateness - not more than 1.0%;
   height - not more than 6.0 mm;
   deviations from an average weight of 20 tablets - deviation from an average weight of individual tablets should not exceed 5%,
   after the tableting press is adjusted, the tableting process is conducted until a tableting mixture is spent in full; at least every 10 minutes, a check of the tableting process is performed for:
      average weight of tablets,
      hardness of tablets.

In the tableting process, tablets are collected into marked containers; upon completion of the process, the containers are closed, representative samples of tablets are taken for conducting an intermediate check according to the specification; then, the **process of applying a film coating onto tablets is conducted which comprises preparation of a film suspension, namely,** a 18% film suspension is used in the process of applying a film coating onto tablets; purified water is stirred so as to form a vortex on the water surface, a dry film coating is gradually added without allowing formation of agglomerates on the solution surface, then a stirring rate is reduced so as to exclude foam formation, and the solution is left for stirring for 45 minutes; after that a **coating is applied** in a coater of a modular plant, e.g. BOSCH Solidlab 1; before starting the coating process, tablets are heated to a temperature of 37-39°C with periodic stirring in the coater drum; when a required tablet temperature is reached, the film suspension spraying is started; the application process is conducted at a product temperature of 35-40°C; the appearance of tablets and a gain in a coating weight are monitored; the coating application process is conducted until obtaining of a required average weight of tablets, including 710.00 mg; when a preset weight of tablets is reached, the process is stopped, and the tablets are cooled to a temperature not higher than 33°C; when the process is complete, the containers are closed, then, representative samples of tablets are taken for intermediate checks, the coated tablets are stored in the closed containers; and then the **tablets are coated** with a coating comprising the pharmaceutical substance of **memantine hydrochloride;** in the process of applying the coating, a 15% film suspension is used, e.g., Opadry 03F180011 or Opadry II 85F200062; purified water stirring is adjusted so as to form a vortex on the water surface; the sifted pharmaceutical substance of memantine hydrochloride is added and stirred until full dissolution under visual monitoring; after that a dry film coating is gradually added without allowing formation of agglomerates on the solution surface, then a stirring rate is reduced so as to exclude foam formation, and the solution is left for stirring for 45 minutes; after that the process of **applying the film coating** is conducted in a coater of a modular plant, e.g., BOSCH Solidlab 1; before starting the coating process, tablets are heated to a temperature of 37-39°C with periodic stirring in the coater drum; when a required tablet temperature is reached, the film suspension spraying is started. The application process is conducted at a product temperature of 35-40°C; the appearance of tablets and a gain in a coating weight are monitored; the coating application process is conducted until obtaining of a required average weight of tablets, including 735.00 mg/760.00 mg; when a preset weight of tablets is reached, the process is stopped, and the tablets are cooled to a temperature not higher than 33°C; when the process is complete, the containers are closed, then, representative samples of tablets are taken for intermediate checks, the coated tablets are stored in the closed containers; and then the **tablets are coated with the final coating,** in the process of applying the film coating, an 18% film suspension is used; purified water stirring is adjusted so as to form a vortex on the water surface, after that a dry film coating is gradually added without allowing formation of agglomerates on the solution surface, then a stirring rate is reduced so as to exclude foam formation, and the solution is left for stirring for 45 minutes; **the coating is applied** in a coater of a modular plant, e.g., BOSCH Solidlab 1; before starting the coating process, tablets are heated to a temperature of 35-45°C with periodic stirring in the coater drum; when a required tablet temperature is reached, the film suspension spraying is started; the application process is conducted at a product temperature of 35-40°C; the appearance of tablets and a gain in a coating weight are monitored; the coating application process is conducted until obtaining of a required average weight of tablets, including 747.00 mg/772.00 mg; when a preset weight of tablets is reached, the process is stopped, and the tablets are cooled to a temperature not higher than 33°C; when the process is complete, the containers are closed, then, representative samples of tablets are taken for an intermediate check according to the specification; the coated tablets are stored in the closed containers; the tablets covered with the film coating **are packed** by placing them, e.g., into PVC/Alu blisters in an automatic blistering machine, such as DPP260ki-2, while the following parameters for forming blister cells being observed:
Pressure - 6 bars;
Formation temperature - 110-120°C;
Sealing parameters:
   Sealing temperature - 130-140°C
   Pressure - 6 bars;
   Rate - 18 cycles/minute;
   Cooling liquid temperature - 10°C;
   after that the blister tightness is checked with the use of, e.g., an Erweka VDT/S tester; the first and last blisters collected are to be checked for tightness.

The comparative study of specific pharmacological activity of the pharmaceutical composition proposed in this invention was conducted by comparison with the citicoline mono-preparation on the model of ischemic stroke in rats. For that purpose, a model of global transient cerebral ischemia with ligation of common carotid arteries was selected on the basis of the Handbook on Conducting Pre-Clinical Studies of Therapeutical Agents, ed. by A.N. Mironov [9]. Long-term memory was evaluated with the use of the Morris Water Maze test (Morris R., "J Neurosci Methods", 1984, Vol. 11, No 1. - P. 47-60 [10]).

**Table. Effect of the preparations under study on long-term memory in rats in the Morris Water Maze test.**

| Preparation | Dose, mg/kg (citicoline + memantine) | Latent period of finding platform, s |
|---|---|---|
| Claimed composition (citicoline + memantine) | 100+2 | 8.7±2.3 |
| | 500+10 | 7.9+1.5 |
| | 1,000+20 | 6.1±1.5 |
| Citicoline | 100+0 | 20.4±7.6 |
| | 500+0 | 13.6±3.5 |
| | 1,000+0 | 10.4±3.5 |
| Memantine | 0+2 | 27.1±7.8 |
| | 0+10 | 14.6±5.2 |
| | 0+20 | 9.2±1.2 |

Unexpectedly, it was found that the supplementing the therapy with memantine having the known indication for therapy of cognitive disorders (dementia) of Alzheimer type caused pronounced neuroprotective and procognitive effects in the correction of cognitive disorders caused by an absolutely different cause, namely, ischemia. The therapy with the preparations under study facilitated successful recall of the acquired habit in the step of long-term memory evaluation. A significant reduction of a latent period (LP) required for finding a target sector (in comparison with animals having an untreated pathology) was noticed in all the groups receiving the pharmaceutical composition proposed in this invention, irrespective of a preparation dose. In the groups receiving citicoline or memantine, the similar effect was observed only in the group receiving the preparation in the maximum studied doses.

**The above examples illustrate, but not limit the use of the formulations and methods of the claimed inventions.**

The developed formulations of the pharmaceutical compositions and the proposed dosage form manufactured according to the described method on the basis of the proposed formulations enable to achieve the technical effects of this invention, i.e. an increase in the therapeutic activity of citicoline or its pharmaceutically acceptable salt due to ensuring its over-cumulative (synergetic) interaction with memantine or its pharmaceutically acceptable salt as well as due to the instantaneous beginning of its action and subsequent effective and prolonged action; simplification of therapy while treating cognitive disorders of vascular origin; the provision of the dosage forms of memantine or its pharmaceutically acceptable salt and citicoline or its pharmaceutically acceptable salt, which are deprived of side effects and are stable in production and storage; the provision of an affordable and inexpensive method for producing dosage forms of memantine or its pharmaceutically acceptable salt and citicoline or its pharmaceutically acceptable salt.

The produced dosage form exhibits stability, including stability in storage, and practical absence of side effects.

Owing to the proposed inventions that form the basis of the dosage forms produced according to the proposed method, the therapeutic effectiveness of the claimed formulation may be increased by means of:
- the modified-release dosage form produced on the basis of the pharmaceutical composition,
- an effective period of action of the dosage form based on the claimed formulation,
- simplification of the scheme of action of the dosage form used.

All the inventions, as discussed herein, are industrially applicable, have passed laboratory trials, are practically feasible and will be of wide use in the pharmaceutical industry; the following is confirmed:
- use of the above pharmaceutical composition,
- use of the proposed dosage form,
- use of the method for producing the proposed dosage form based on the pharmaceutical composition.

Each of the proposed inventions is industrially applicable in the field of pharmaceutics, ensures stability of the claimed properties, said stability being maintained for a period not less than 3 years.

Use of the proposed inventions expands the range of dosage forms.

Furthermore, the proposed method for producing the dosage form ensures stability of the dosage form properties, said stability of the properties being maintained for a period not less than 3 years.

Thus, the expected technical effect is achieved by the proposed group of inventions, including the pharmaceutical composition having neuromodulatory, antiparkinsonism, neuroprotective, and antispastic effects, intended for therapy of cognitive disorders of vascular origin, slowing down and inhibiting glutamatergic neurotransmission and progress of neurodegenerative processes,
- by the pharmaceutical composition providing an increase in the therapeutic activity of the claimed quantitative and qualitative formulation of the ingredients owing to the instantaneous beginning of action and effective and prolonged action of the formulation of the ingredients in therapy of cognitive disorders of vascular origin, having neuromodulatory, antiparkinsonism, neuroprotective, and antispastic effects, slowing down and inhibiting glutamatergic neurotransmission and progress of neurodegenerative processes,
- by the dosage form which enables, when the above pharmaceutical formulation is administered, to simplify the scheme of action of the claimed quantitative and qualitative formulation of the ingredients in therapy of cognitive disorders of vascular origin, and has neuromodulatory, antiparkinsonism, neuroprotective, and antispastic effects, slows down and inhibits glutamatergic neurotransmission and progress of neurodegenerative processes,
- and by the method for producing the above dosage form based on the proposed pharmaceutical composition, which is intended for producing tablets used in therapy of cognitive disorders of vascular origin, has neuromodulatory, antiparkinsonism, neuroprotective, and antispastic effects, slows down and inhibits glutamatergic neurotransmission and progress of neurodegenerative processes.

Furthermore, a technical effect is an expansion of the range of dosage forms intended for therapy of cognitive disorders of vascular origin, having neuromodulatory, antiparkinsonism, neuroprotective, and antispastic effects, slowing down and inhibiting glutamatergic neurotransmission and progress of neurodegenerative processes.

The proposed group of inventions is industrially applicable and may be practiced, which is evidenced by the presented exemplary embodiments of the group of inventions which will be of wide use both in Russia and foreign countries for producing pharmaceutical compositions and dosage forms having neuromodulatory, antiparkinsonism, neuroprotective, and antispastic effects, slowing down and inhibiting glutamatergic neurotransmission and progress of neurodegenerative processes, and intended for therapy of cognitive disorders of vascular origin.

### INFORMATION SOURCES:

1. Patent RU2429851 "COMPOSITIONS CONTAINING PUFA AND/OR URIDINE, AND METHODS OF APPLICATION THEREOF", patent holder: MASSACHUSETTS INSTITUTE OF TECHNOLOGY (US), publ.: 27.09.2011, IPC: A61K 31/685, A61P 25/28.
2. Patent RU2603470 "METHOD AND PHARMACEUTICAL COMPOSITION FOR TREATING ALZHEIMER'S DISEASE AND DISORDERS, DISTURBED OR LOST MEMORY", patent holder: MASSACHUSETTS INSTITUTE OF TECHNOLOGY (US), publ.: 27.11.2016, IPC: A61K 31/685, A61P 25/28.
3. Patent RU2605339 "AGENT FOR REDUCTION OF GLUTAMATE-INDUCED APOPTOSIS AND INHIBITION OF NMDA-RECEPTOR, POSSESSING IMMUNOMODULATORY ACTION, FOR TREATING NERVOUS SYSTEM DISORDERS, EFFECTS OF CRANIOCEREBERAL INJURY AND ISCHEMIC AND HEMORRHAGIC STROKE", patent holder: Closed Joint-Stock Society "EcoPharmPlus" (RU), publ.: 20.12.2016, IPC: A61K 31/7068, A61K 31/13, A61K 47/48, A61P 9/10, A61P 25/00.
4. Patent RU2371173 "MEMANTINE THERAPY FOR ALZHEIMER'S DISEASE OF LOW AND LOW TO MODERATE SEVERITY", patent holder: "MERZ PHARMA GMBH & CO. KGAA" (DE), publ.: 27.10.2009, IPC: A61K 31/13, A61P 25/28.
5. Patent RU2326660 "ORAL PHARMACEUTICAL COMPOSITION MEMANTINE (OPTIONS) AND METHOD OF PREPARATION (OPTIONS)", patent holder: Closed Joint-Stock Society "Biological Research and Systems" (RU), publ.: 20.06.2008, IPC: A61K 31/13, A61K 9/48, A61P 25/28.
6. Patent RU2404750 "COMPOSITION CONTAINING BASE OR COAT FOR MODERATED RELEASE AND ANTAGONIST OF NMDA RECEPTOR, METHOD FOR INTRODUCTION OF SUCH NMDA ANTAGONIST TO INDIVIDUAL", patent holder: ADAMAS PHARMACEUTICALS, INC. (US), publ.: 27.11.2010, IPC: A61K 9/20, A61P 25/28.
7. Patent RU2483715 "SOLID DOSAGE FORM OF PREPARATIONS OF MEMANTINE AND ITS SALTS", patent holder: Limited Liability Company "ACADEMPHARM, LLC" (RU), publ.: 10.06.2013, IPC: A61K 31/13, A61K 47/38, A61K 9/16, A61K 9/20, A61P 25/16.
8. Patent RU2390354 "SLOW-RELEASE MATRIX PREPARATION CONTAINING BASE AGENT OR ITS SALT AND METHOD FOR MAKING THEREOF", patent holder: EISAI R&D MANAGEMENT CO., LTD (JP), publ.: 27.05.2010, IPC: A61K 47/32, A61K 9/26, A61K 31/445, A61J 3/10.
9. Handbook on Conducting Pre-Clinical Studies of Therapeutic Agents, ed. by A.N. Mironov. Part I, ed. by A.N. Mironov - M.: "Grif and K", 2012. - 944 pp.
10. Morris R. Developments of a water-maze procedure for studying spatial learning in the rat // J Neurosci Methods. - 1984. - Vol. 11, No 1. - pp. 47-60.

## Claims

1. A pharmaceutical composition having neuromodulatory, antiparkinsonism, neuroprotective, and antispastic effects, intended for therapy of cognitive disorders of vascular origin, slowing down and inhibiting glutamatergic neurotransmission and progress of neurodegenerative processes, comprising memantine or its pharmaceutically acceptable salt in the range from 5 to 20 mg and citicoline or its pharmaceutically acceptable salt in the range from 450 to 550 mg.

2. The pharmaceutical composition of Claim 1, **characterized in that** the pharmaceutically acceptable salt of memantine is memantine hydrochloride, and the pharmaceutically acceptable salt of citicoline is citicoline monosodium salt.

3. A pharmaceutical composition having neuromodulatory, antiparkinsonism, neuroprotective, and antispastic effects, intended for therapy of cognitive disorders of vascular origin, slowing down and inhibiting glutamatergic neurotransmission and progress of neurodegenerative processes, comprising memantine hydrochloride in the range from 5 tp 20 mg and the citicoline monosodium salt in the range from 450 to 550 mg, the ratio of quantitative and qualitative formulation of ingredients ot the pharmaceutical composition being as follows, wt%:
| | |
|---|---|
| Citicoline monosodium salt | 65.95-69.95 |
| Memantine hydrochloride | 0.67-2.50 |
| Microcrystalline cellulose | 0.63-5.13 |
| Hydroxypropyl methylcellulose having molecular weight of 1,000,000 | 17.23-17.80 |
| Colloidal silica | 0.91-1.0 |
| Magnesium stearate | 0.91-1.0 |
| Film coating I | 3.00-7.00 |
| Film coating II | 1.00-2.00 |

4. The pharmaceutical composition of Claim 3, **characterized in that** the film coating I is produced from a mixture of hydroxypropyl methylcellulose, titanium dioxide, and macrogol.

5. The pharmaceutical composition of Claim 3, **characterized in that** the film coating II is produced from a mixture of polyvinyl alcohol, talc, macrogol, iron oxides, and titanium dioxide.

6. A pharmaceutical composition having neuromodulatory, antiparkinsonism, neuroprotective, and antispastic effects, intended for therapy of cognitive disorders of vascular origin, slowing down and inhibiting glutamatergic neurotransmission and progress of neurodegenerative processes, comprising memantine hydrochloride in the range from 5 to 20 mg and citicoline monosodium salt in the range from 450 to 550 mg, the ratio of quantitative and qualitative formulation of ingredients of the pharmaceutical composition being as follows, mg:
| | |
|---|---|
| Citicoline monosodium salt | 450-550 |
| Memantine hydrochloride | 5-20 |
| Microcrystalline cellulose | 5-35 |
| Hydroxypropyl methylcellulose having molecular weight of 1,000,000 | 120.5-140.6 |
| Colloidal silica | 6.8-7.2 |
| Magnesium stearate | 6.8-7.2 |
| Film coating I | 36.4-41.5 |
| Film coating II | 21.8-24.5 |

7. The pharmaceutical composition of Claim 5, **characterized in that** the film coating I is produced from a mixture of hydroxypropyl methylcellulose, titanium dioxide, and macrogol.

8. The pharmaceutical composition of Claim 5, **characterized in that** the film coating II is produced from a mixture of polyvinyl alcohol, talc, macrogol, iron oxides, and titanium dioxide.

9. A solid oral dosage form having neuromodulatory, antiparkinsonism, neuroprotective, and antispastic effects, intended for therapy of cognitive disorders of vascular origin, slowing down and inhibiting glutamatergic neurotransmission and progress of neurodegenerative processes, comprising, as the active substances, memantine or its pharmaceutically acceptable salt in the range from 5.00 to 20.00 mg and citicoline or its pharmaceutically acceptable salt in the range from 450 to 550 mg, **characterized in that** the dosage form is manufactured in the form of a modified release tablet coated with a film coating.

10. A solid oral dosage form having neuromodulatory, antiparkinsonism, neuroprotective, and antispastic effects, intended for therapy of cognitive disorders of vascular origin, slowing down and inhibiting glutamatergic neurotransmission and progress of neurodegenerative processes, comprising, as the active substances, memantine hydrochloride in the range from 5.00 to 20.00 mg and citicoline monosodium salt in the range from 450 to 550 mg, **characterized in that** the dosage form is manufactured in the form of a modified-release tablet coated with a film coating, the ratio of quantitative and qualitative formulation of the ingredients, per 1 tablet, being as follows, wt%:
| | |
|---|---|
| Citicoline monosodium salt | 65.95-69.95 |
| Memantine hydrochloride | 0.67-2.50 |
| Microcrystalline cellulose | 0.63-5.13 |
| Hydroxypropyl methylcellulose having molecular weight of 1,000,000 | 17.23-17.80 |
| Colloidal silica | 0.91-1.0 |
| Magnesium stearate | 0.91-1.0 |
| Film coating I | 5.18-5.33 |
| Film coating II | 2.85-3.2 |

11. The solid oral dosage form of Claim 10, **characterized in that** the film coating I is produced from a mixture of hydroxypropyl methylcellulose, titanium dioxide, and macrogol.

12. The solid oral dosage form of Claim 10, **characterized in that** the film coating II is produced from a mixture of polyvinyl alcohol, talc, macrogol, iron oxides, and titanium dioxide.

13. A solid oral dosage form, having neuromodulatory, antiparkinsonism, neuroprotective, and antispastic effects, intended for therapy of cognitive disorders of vascular origin, slowing down and inhibiting glutamatergic neurotransmission and progress of neurodegenerative processes, comprising, as the active substances, memantine hydrochloride in the range from 5.00 to 20.00 mg and citicoline monosodium salt in the range from 450 to 550 mg, **characterized in that** the dosage form is manufactured in the form of a modified-release tablet coated with a film coating, the ratio of quantitative and qualitative formulation of the ingredients, per 1 tablet, being as follows, mg.:
| | |
|---|---|
| Citicoline monosodium salt | 450-550 |
| Memantine hydrochloride | 5-20 |
| Microcrystalline cellulose | 5-35 |
| Hydroxypropyl methylcellulose having molecular weight of 1,000,000 | 120.5-140.6 |
| Colloidal silica | 6.8-7.2 |
| Magnesium stearate | 6.8-7.2 |
| Film coating I | 36.4-41.5 |
| Film coating II | 21.8-24.5 |

14. The solid oral dosage form of Claim 13, **characterized in that** the film coating I is produced from a mixture of hydroxypropyl methylcellulose, titanium dioxide, and macrogol.

15. The solid oral dosage form of Claim 13, **characterized in that** the film coating II is produced from a mixture of polyvinyl alcohol, talc, macrogol, iron oxides, and titanium dioxide.

16. A method for producing the solid oral dosage form of any one of Claims 9-15, **characterized in that** magnesium stearate, the citicoline monosodium salt, memantine hydrochloride, microcrystalline cellulose, hydroxypropyl methylcellulose, and colloidal silica are sifted through a sieve with a mesh of not more than 0.5 mm, then are loaded into a mixer-granulator or a similar mixing device; citicoline monosodium salt, microcrystalline cellulose and hydroxypropyl methylcellulose are mixed to a homogenous state at a mixing rate of 160-240 rpm for a period from 2 to 10 minutes; after that the mixture is moistened with purified water, while continuously stirring the mixture at a stirring rate of 150-400 rpm for not more than 5 minutes, for producing a granulated material; after that, the produced granulated material is dried in the fluidized bed conditions, while maintaining the following parameters: an input air temperature - 45-65°C, residual moisture of the granulated material from 2.5 to 4.5%; then, the obtained mixture is dusted with colloidal silica and magnesium stearate; further, the obtained mixture is tableted in a rotary tableting press for forming tablet cores with the use of tablet tooling having a diameter from 10 to 15 mm, the tableting process being monitored for the following parameters: hardness - 150-290 H, grateness - not more than 1.0%, height - not more than 7.0 mm; then, a film coating I, which is produced from a mixture of hydroxypropyl methylcellulose, titanium dioxide, and macrogol, is applied onto the manufactured tablet cores; in the process of dissolving the film coating I, a pharmaceutical substance of memantine hydrochloride is added in portions and mixed until formation of a homogenous suspension, after that, the coating thus obtained is applied onto the tablet cores at 35-40°C with periodic stirring; then, a solution of a film coating II is prepared from a mixture of polyvinyl alcohol, talc, macrogol, iron oxides, and titanium dioxide and applied onto the tablets in the same conditions as used for the film coating I.

17. The method of Claim 16, **characterized in that** microcrystalline cellulose of 101 grade is used.

18. The method of Claim 16, **characterized in that** colloidal silica of A 200 grade is used.

19. The method of Claim 16, **characterized in that** the film coating I is applied onto a tablet core in the coater drum.

20. A method of producing a dosage form based on the pharmaceutical composition described in any one of Claims 1-8, **characterized in that** magnesium stearate, citicoline monosodium salt, memantine hydrochloride, microcrystalline cellulose, hydroxypropyl methylcellulose, and colloidal silica are sifted through a sieve with a mesh of not more than 0.5 mm; then the citicoline monosodium salt, microcrystalline cellulose and hydroxypropyl methylcellulose are loaded into a mixer-granulator or a similar mixing device and mixed to a homogenous state at a mixing rate of 160-240 rpm for a period from 2 to 10 minutes; after that the mixture is moistened with purified water, while continuously stirring the mixture at a stirring rate of 150-400 rpm for not more than 5 minutes, for producing a granulated material; after that, the produced granulated material is dried in the fluidized bed conditions, while maintaining the following parameters: an input air temperature - 45-65°C, residual moisture of the granulated material - 2.5 to 4.5%; then, the produced granulated material is sifted through a sieve having a mesh diameter of not more than 1.0 mm; then, the obtained mixture is dusted with colloidal silica for 3 to 5 minutes; then a portion of hydroxypropyl methylcellulose is added to the mixture; stirring is continued for another 8 to 10 minutes; then magnesium stearate is added, and stirring is continued for another 2-3 minutes; thus, the mixture for tableting is prepared; then, the mixture is tableted in a rotary tableting press for forming tablet cores with the use of a tablet tooling having a diameter from 10 to 15 mm, the tableting process being monitored for the following parameters: hardness - 150-290 H, grateness - not more than 1.0%, height - not more than 7.0 mm; after that, a film coating I is applied to the formed tablet cores; in the process of dissolving the film coating I, a pharmaceutical substance of memantine hydrochloride is added in portions and mixed until formation of a homogenous suspension; after that, the film coating I thus produced is applied onto the tablet core at a tablet core temperature of 35-40°C with periodic stirring; then, a solution of a film coating II is prepared from a mixture of polyvinyl alcohol, talc, macrogol, iron oxides, and titanium dioxide; and it is applied to the tablets in the same conditions as used for the film coating I, the quantitative and qualitative formulation of the ingredients of the dosage form per 1 tablet being as follows, wt%:
| | |
|---|---|
| Citicoline monosodium salt | 65.95-69.95 |
| Memantine hydrochloride | 0.67-2.50 |
| Microcrystalline cellulose | 0.63-5.13 |
| Hydroxypropyl methylcellulose having molecular weight of 1,000,000 | 17.23-17.80 |
| Colloidal silica | 0.91-1.0 |
| Magnesium stearate | 0.91-1.0 |
| Film coating I | 5.18-5.33 |
| Film coating II | 2.85-3.2 |

21. The method of Claim 20, **characterized in that** the film coating I is applied onto the tablet core in the coater drum.

22. The method of Claim 20, **characterized in that** microcrystalline cellulose of 101 grade is used as microcrystalline cellulose.

23. The method of Claim 20, **characterized in that** colloidal silica of A 200 grade is used as colloidal silica.

24. A method for producing the dosage form based on the pharmaceutical composition described in any one of Claims 1-8, **characterized in that** magnesium stearate, the citicoline monosodium salt, memantine hydrochloride, microcrystalline cellulose, hydroxypropyl methylcellulose, and colloidal silica are sifted through a sieve having a mesh diameter of not more than 0.5 mm; then, the citicoline monosodium salt, microcrystalline cellulose and hydroxypropyl methylcellulose are loaded into a mixer-granulator or a similar mixing device and mixed to a homogenous state at a mixing rate of 160-240 rpm for a period from 2 to 10 minutes; and after that the mixture is moistened with purified water, while continuously stirring the mixture at a stirring rate of 150-400 rpm for not more than 5 minutes, for producing a granulated material; after that, the produced granulated material is dried in the fluidized bed conditions, while maintaining the following parameters: an input air temperature - 45-65°C, residual moisture of the granulated material - 2.5 to 4.5%; the produced granulated material is sifted through a sieve having a mesh diameter of not more than 1.0 mm; then, the obtained mixture is dusted with colloidal silica for 3 to 5 minutes; then a portion of hydroxypropyl methylcellulose is added to the mixture; stirring is continued for another 8 to 10 minutes, then magnesium stearate is added, and stirring is continued for another 2-3 minutes; thus, the mixture for tableting is prepared; then, the mixture is tableted in a rotary tableting press for forming tablet cores with the use of a tablet tooling having a diameter from 10 to 15 mm, the tableting process being monitored for the following parameters: hardness - 150-290 H, grateness - not more than 1.0%, height - not more than 7.0 mm; after that, a film coating I is applied to the formed tablet cores; in the process of dissolving the film coating I, a pharmaceutical substance of memantine hydrochloride is added in portions and mixed until formation of a homogenous suspension; after that, the film coating I thus produced is applied at a tablet core temperature of 35-40°C with periodic stirring; then, a solution of a film coating II is prepared from a mixture of polyvinyl alcohol, talc, macrogol, iron oxides, and titanium dioxide; and it is applied to the tablets in the same conditions as used for the film coating I, the quantitative and qualitative formulation of the ingredients of the dosage form per 1 tablet being as follows, mg:
| | |
|---|---|
| Citicoline monosodium salt | 450-550 |
| Memantine hydrochloride | 5-20 |
| Microcrystalline cellulose | 5-35 |
| Hydroxypropyl methylcellulose having molecular weight of 1,000,000 | 120.5-140.6 |
| Colloidal silica | 6.8-7.2 |
| Magnesium stearate | 6.8-7.2 |
| Film coating I | 36.4-41.5 |
| Film coating II | 21.8-24.5 |

25. The method of Claim 24, **characterized in that** microcrystalline cellulose of 101 grade is used as microcrystalline cellulose.

26. The method of Claim 24, **characterized in that** colloidal silica of A 200 grade is used as colloidal silica.

27. A drug product in the form of a modified-release tablet, comprising, as the active substances, the citicoline monosodium salt and memantine hydrochloride, microcrystalline cellulose as the excipient, hypromellose as the matrix agent, colloidal silica as the glidant, magnesium stearate as the lubricant, a film coating as the coating and carrier, the quantitative and qualitative formulation of the ingredients of the dosage form per 1 tablet being as follows, wt%:
| | |
|---|---|
| Citicoline monosodium salt | 69.95 |
| (equivalent to citicoline base) | (66.93) |
| Microcrystalline cellulose 101 | 4.08 |
| Hypromellose of 2208 type (K100M) | 17.80 |
| Colloidal silica A 200 | 0.94 |
| Magnesium stearate | 0.94 |
| Film coating I | 1.34 |
| Memantine hydrochloride | 0.67 |
| Film coating I | 2.68 |
| Film coating II | 1.61 |

28. The drug product of Claim 27, **characterized in that** the film coating I is produced from a mixture of hydroxypropyl methylcellulose, titanium dioxide, and macrogol.

29. The drug product of Claim 27, **characterized in that** the film coating II is produced from a mixture of polyvinyl alcohol, talc, macrogol, iron oxides, and titanium dioxide.

30. A drug product in the form of a modified-release tablet, comprising, as the active substances, citicoline sodium and memantine hydrochloride, microcrystalline cellulose as the excipient, hypromellose as the matrix agent, colloidal silica as the glidant, magnesium stearate as the lubricant, a film coating as the coating and carrier, the quantitative and qualitative formulation of the ingredients of the dosage form per 1 tablet being as follows, mg:
| | |
|---|---|
| Citicoline monosodium salt | 522.50 |
| (equivalent to citicoline base) | (500.00) |
| Microcrystalline cellulose 101 | 30.50 |
| Hypromellose of 2208 type (K100M) | 133.00 |
| Colloidal silica A 200 | 7.00 |
| Magnesium stearate | 7.00 |
| Film coating I | 10.00 |
| Memantine hydrochloride | 5.00 |
| Film coating I | 20.00 |
| Film coating II | 12.00 |

31. The drug product of Claim 30, **characterized in that** the film coating I is produced from a mixture of hydroxypropyl methylcellulose, titanium dioxide, and macrogol.

32. The drug product of Claim 30, **characterized in that** the film coating II is produced from a mixture of polyvinyl alcohol, talc, macrogol, iron oxides, and titanium dioxide.

33. A drug product in the form of a modified-release tablet, comprising, as the active substances, citicoline sodium and memantine hydrochloride, microcrystalline cellulose as the excipient, hypromellose as the matrix agent, colloidal silica as the glidant, magnesium stearate as the lubricant, a film coating as the coating and carrier, the quantitative and qualitative formulation of the ingredients of the dosage form per 1 tablet being as follows, wt%:
| | |
|---|---|
| Citicoline monosodium salt | 67.68 |
| (equivalent to citicoline base) | (64.77) |
| Microcrystalline cellulose 101 | 3. 95 |
| Hypromellose of 2208 type (K100M) | 17.23 |
| Colloidal silica A 200 | 0.91 |
| Magnesium stearate | 0.91 |
| Film coating I | 1.30 |
| Memantine hydrochloride | 1.30 |
| Film coating I | 5.18 |
| Film coating II | 1.55 |

34. The drug product of Claim 33, **characterized in that** the film coating I is produced from a mixture of hydroxypropyl methylcellulose, titanium dioxide, and macrogol.

35. The drug product of Claim 33, **characterized in that** the film coating II is produced from a mixture of polyvinyl alcohol, talc, macrogol, iron oxides, and titanium dioxide.

36. A drug product in the form of a modified-release tablet, comprising sodium citicoline and memantine hydrochloride as the pharmaceutical substances, microcrystalline cellulose as the excipient, hypromellose as the matrix agent, colloidal silica as the glidant, magnesium stearate as the lubricant, a film coating as the coating and carrier, the quantitative and qualitative formulation of the ingredients of the dosage form per 1 tablet being as follows, mg:
| | |
|---|---|
| Citicoline monosodium salt | (522.50) |
| (equivalent to citicoline base) | (500.00) |
| Microcrystalline cellulose 101 | 30.50 |
| Hypromellose of 2208 type (K100M) | 133.00 |
| Colloidal silica A 200 | 7.00 |
| Magnesium stearate | 7.00 |
| Film coating I | 10.00 |
| Memantine hydrochloride | 10.00 |
| Film coating I | 40.00 |
| Film coating II | 12.00 |

37. The drug product of Claim 36, **characterized in that** the film coating I is produced from a mixture of hydroxypropyl methylcellulose, titanium dioxide, and macrogol.

38. The drug product of Claim 36, **characterized in that** the film coating II is produced from a mixture of polyvinyl alcohol, talc, macrogol, iron oxides, and titanium dioxide.

39. Use of the pharmaceutical composition of any one of Claims 1-8 for therapy of cognitive disorders of vascular origin.

40. Use of the solid oral dosage form of any one of Claims 9-15 for therapy of cognitive disorders of vascular origin.

41. Use of the method of any one of Claims 16-26 for producing the dosage form for therapy of cognitive disorders of vascular origin.
